# EUROPEAN PATENT APPLICATION

(11) **EP 2 777 811 A1**
(43) Date of publication of application: **17.09.2014**
(21) Application number: 14159134.7
(22) Date of filing: 12.03.2014
(51) Int. Cl.: B01L 7/00, G01N 35/00, G01N 35/10

(54) **Nucleic acid amplification reaction device and nucleic acid amplification method**

(30) Priority: 13.03.2013 JP 2013050652
(71) Applicant: Seiko Epson Corporation, Shinjuku-ku Tokyo (JP)
(72) Inventor: Koeda, Hiroshi, Nagano, 392-8502 (JP); Takagi, Fumio, Nagano, 392-8502 (JP)
(74) Representative: HOFFMANN EITLE

(57) **Abstract**

A nucleic acid amplification reaction device (50) includes a rotary body (61) having a mounting portion (62) on which a cartridge (1), which includes a tube (20) that has a plug (47) containing an eluate in which a nucleic acid having bound to nucleic acid-binding solid-phase carriers is eluted from the carriers and a nucleic acid amplification reaction container (30) that is in communication with the tube and contains oil (48) undergoing phase separation from the eluate, is mounted; and a heater forming a temperature gradient (65B, 65C) inside the nucleic acid amplification reaction container, in which when the posture of the cartridge is changed due to the rotation of the rotary body, a droplet of the eluate (47) introduced from the tube (20) moves inside the nucleic acid amplification reaction container (30).

## Description

### BACKGROUND

### 1. Technical Field

The present invention relates to a nucleic acid amplification reaction device and a nucleic acid amplification method.

### 2. Related Art

JP-A-2009-136250 and JP-A-2012-115208 disclose a device conducting thermal cycling by rotating a biochip filled with a reaction solution and oil, which undergoes phase separation from the reaction solution and has a specific gravity smaller than that of the reaction solution, such that the reaction solution moves inside the biochip. However, in JP-A-2009-136250 and JP-A-2012-115208, the process for making the reaction solution sealed in (dispensed into) the inside of the biochip is not taken into consideration.

### SUMMARY

An advantage of some aspects of the invention is that it provides a novel nucleic acid amplification reaction device and a nucleic acid amplification method.

An aspect of the invention is directed to a nucleic acid amplification reaction device including a rotary body having a mounting portion on which a cartridge, which includes a tube that has a plug containing an eluate in which a nucleic acid having bound to nucleic acid-binding solid-phase carriers is eluted from the carriers and a nucleic acid amplification reaction container that is in communication with the tube and contains oil undergoing phase separation from the eluate, is mounted, and a heater forming a temperature gradient inside the nucleic acid amplification reaction container, in which when the posture of the cartridge is changed due to the rotation of the rotary body, a droplet of the eluate introduced from the tube moves inside the nucleic acid amplification reaction container.

Other characteristics of the aspect of the invention will be further clarified by the description of the present specification and the attached drawings.

### BRIEF DESCRIPTION OF THE DRAWINGS

Figs. 1A and 1B are views for illustrating a cartridge.
Figs. 2A to 2C are views for illustrating the operation of the cartridge.
Figs. 3A to 3D are views for illustrating a tank.
Fig. 4 is a view for illustrating a fixing claw, a guide plate, and a mounting portion.
Figs. 5A and 5B are views for illustrating the periphery of a PCR container.
Fig. 6A is a perspective view of the internal constituents of a PCR device. Fig. 6B is a lateral view of main constituents of the PCR device.
Fig. 7 is a block diagram of the PCR device.
Fig. 8A is a view for illustrating a rotary body. Fig. 8B is a view for illustrating a state where the cartridge has been mounted on the mounting portion of the rotary body.
Figs. 9A to 9D are views for illustrating the state of the PCR device at the time when the cartridge is mounted on the device.
Fig. 10 is a view schematically showing the behavior of magnetic beads at the time when magnets are moved downward.
Figs. 11A to 11C are views for illustrating nucleic acid elution process.
Fig. 12 is a view schematically showing the behavior of the magnetic beads at the time when the magnets oscillate.
Fig. 13 is a table showing whether or not the magnets oscillate.
Figs. 14A to 14C are views for illustrating solution droplet formation process.
Figs. 15A and 15B are views for illustrating thermal cycling process.
Figs. 16A and 16B are views for illustrating the cartridge of a second embodiment.
Fig. 17A is a view for illustrating the initial state of the cartridge of the second embodiment. Fig. 17B is a lateral view showing a state where a plunger is pushed in the state described in FIG. 17A such that a seal comes into contact with a lower syringe. Fig. 17C is a view for illustrating the cartridge of the second embodiment in which the plunger has been pushed.
Figs. 18A and 18B are views for illustrating the periphery of PCR container of the second embodiment.
Fig. 19 is a view showing a nucleic acid extraction kit used in an example and a device obtained by assembling the kit.
Fig. 20 is a graph showing results of real time PCR in an example.

### DESCRIPTION OF EXEMPLARY EMBODIMENTS

By the description of the present specification and the attached drawings, at least the following matters are clarified.

The description clarifies a nucleic acid amplification reaction device including a rotary body having a mounting portion on which a cartridge, which includes a tube that has a plug containing an eluate in which a nucleic acid having bound to nucleic acid-binding solid-phase carriers is eluted from the carriers and a nucleic acid amplification reaction container that is in communication with the tube and contains oil undergoing phase separation from the eluate, is mounted, and a heater forming a temperature gradient inside the nucleic acid amplification reaction container, in which when the posture of the cartridge is changed due to the rotation of the rotary body, a droplet of the eluate introduced from the tube moves inside the nucleic acid amplification reaction container.

According to this device, the posture of both the tubes, in which nucleic acid elution process is performed, and the nucleic acid amplification reaction container is changed at the same time. Therefore, the time taken for the process can be shortened.

It is desirable for the rotary shaft of the rotary body to be positioned not at the side of the nucleic acid amplification reaction container but at the side of the tube. If the rotary shaft is positioned in this manner, the rotary body can be miniaturized.

It is desirable for the mounting portion to have a tube-fixing portion for fixing the tube and a container-fixing portion for fixing the nucleic acid amplification reaction container. If the mounting portion has these portions, the cartridge is stably fixed.

It is desirable for the heater to be disposed in the rotary body. If the heater is disposed in this manner, the temperature gradient formed inside the nucleic acid amplification reaction container is stabilized.

It is desirable for the nucleic acid amplification reaction device to have a heater for elution which heats the plug of the cartridge mounted on the mounting portion. If the device has the heater, liberation of nucleic acid from the nucleic acid-binding solid-phase carriers is accelerated.

It is desirable for the nucleic acid amplification reaction device to further have a magnet-moving mechanism that moves a magnet along the tube. If the device has the mechanism, the nucleic acid-binding solid-phase carriers become movable inside the tube by the magnet-moving mechanism.

It is desirable for the magnet-moving mechanism to change a distance between the magnet and the tube by causing the magnet to oscillate. In this manner, it is possible to regulate the aggregation and diffusion of the nucleic acid-binding solid-phase carriers that are caused inside the tube.

It is desirable for the nucleic acid amplification reaction device to further have a pushing mechanism pushing a plunger that is disposed in the cartridge to push out liquid from the tube to the nucleic acid amplification reaction container. If the device has the pushing mechanism, liquid in the tube can be introduced into the nucleic acid amplification reaction container by the pushing mechanism.

The description clarifies a nucleic acid amplification method including mounting a cartridge, which includes a tube that has a plug containing an eluate in which nucleic acid having bound to nucleic acid-binding solid-phase carriers is eluted from the carriers and a nucleic acid amplification reaction container that is in communication with the tube and contains oil undergoing phase separation from the eluate, on a mounting portion, and forming a temperature gradient inside the nucleic acid amplification reaction container and changing the posture of the cartridge by causing a rotary body having the mounting portion to rotate such that a droplet of the eluate introduced from the tube moves inside the nucleic acid amplification reaction container.

According to the above method, the posture of the tube, in which nucleic acid elution process is performed, and the posture of the nucleic acid amplification reaction container are changed at the same time. Therefore, the time taken for the process can be shortened.

### First embodiment

First, the cartridge to be mounted on a PCR device 50 (nucleic acid amplification reaction device) will be described, and then the constitution and operation of the PCR device 50 of the present embodiment will be described.

### Cartridge 1

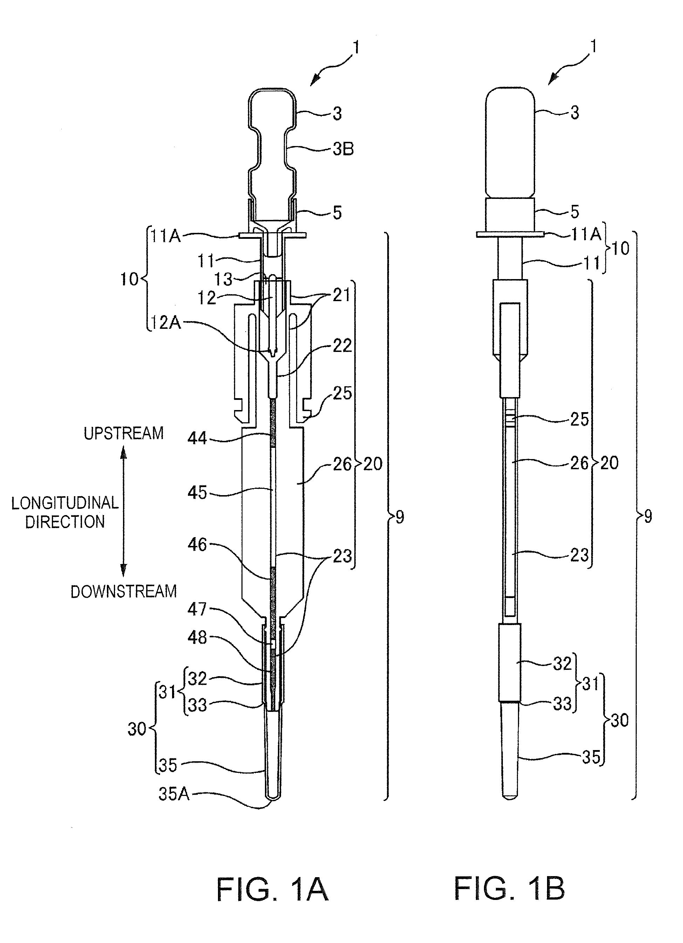

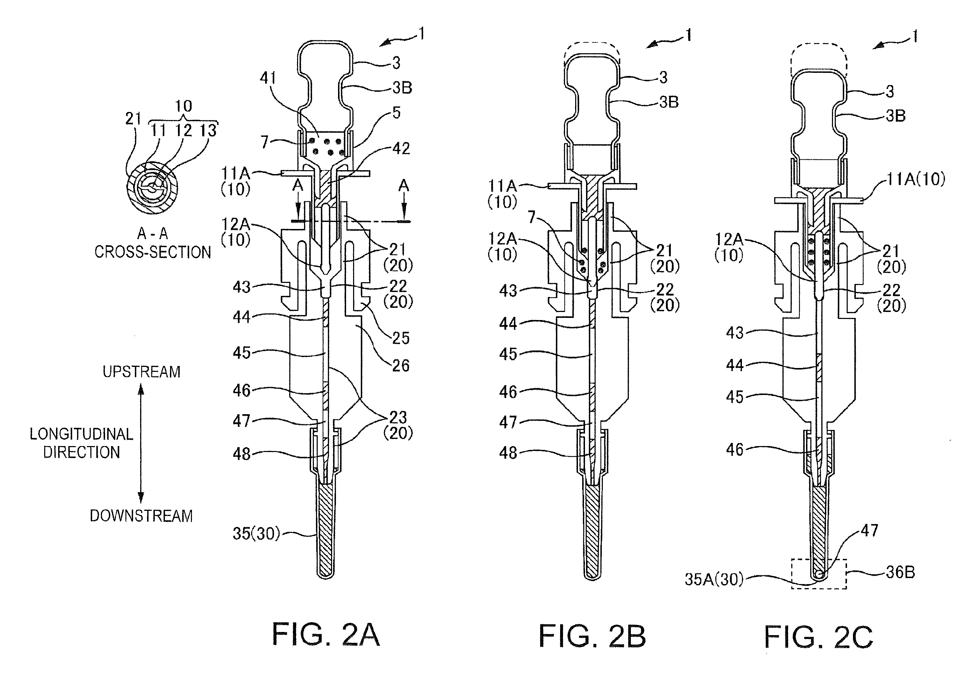

Figs. 1A and 1B are views for illustrating a cartridge 1. Figs. 2A to 2C are views for illustrating the operation of the cartridge 1. Fig. 2A is a view for illustrating the initial state of the cartridge 1. Fig. 2B is a lateral view showing a state where a plunger 10 is pushed in the state described in FIG. 2A such that a seal 12A comes into contact with a lower syringe 22. Fig. 2C is a view for illustrating the cartridge 1 in which the plunger 10 has been pushed.

The cartridge 1 is a container in which nucleic acid elution process for causing nucleic acid to be eluted from magnetic beads 7 having bound to the nucleic acid is conducted. The cartridge 1 is also a container in which thermal cycling process for causing a polymerization reaction of a reaction solution 47 to be a PCR solution is conducted.

The nucleic acid extraction process is performed in a tank 3, and the nucleic acid is purified while passing through a tube 20. The material of the tube 20 is not particularly limited, and the tube can be made of, for example, glass, resins such as plastic, and metals. Particularly, it is preferable to select transparent glass or resin as the material of the tube 20, since the inside of the tube 20 can be observed from the outside. Moreover, it is preferable to select materials that transmits magnetic force or nonmagnetic materials as the material of the tube 20, since this makes it easy to move magnetic particles by applying magnetic force from the outside of the tube 20 when the magnetic particles are passed through the tube 20. Furthermore, it is preferable for the material of the tube to be heat resistant to a temperature of at least 100°C or higher since a heater (a heater for elution 65A or a high temperature-side heater 65B which will be described later) is disposed near the tube. In addition, the tube 20 and the tank may be made of the same material.

The tube 20 has a washing solution plug 45, a reaction solution plug 47, and an oil plug. Since the magnetic beads 7 having bound to the nucleic acid are attracted to a magnet of the outside, if the magnet is moved at the outside along the tube 20, the magnetic beads 7 move inside the tube 20, pass through the washing solution plug 45, and reach the reaction solution plug 47. The nucleic acid having bound to the magnetic beads 7 is washed with the washing solution in the washing solution plug 45 and eluted in the reaction solution plug 47. The "plug" refers to a certain liquid that takes up a section inside the tube 20. For example, in Figs. 2A to 2C, the liquid held in the form of a column inside a capillary 23 is called a "plug". Oil undergoes phase separation from other liquid (oil is not mixed with other liquids). Therefore, a plug formed of the oil functions to prevent the plugs at both sides of the plug from being mixed with each other. It is preferable for air bubbles or other liquids not to be present in a plug or between plugs. However, as long as the magnetic beads 7 can pass through the plug, air bubbles or other liquids may be present.

The type of oil is not particularly limited, and mineral oil, silicone oil (such as 2CS silicone oil), plant oil, and the like can be used. However, if oil having a higher viscosity is used, when the nucleic acid-binding solid-phase carriers are moved in the interface between the oil and the upper plug, a "wiping effect" produced by the oil can be enhanced. Consequently, when the nucleic acid-binding solid-phase carriers are moved from the upper plug to the plug formed of the oil, it is possible to make it more difficult for water-soluble components, which have adhered to the nucleic acid-binding solid-phase carriers, to be mixed into the oil.

The thermal cycling process is performed in the PCR container 30 of the cartridge 1. The PCR container 30 is filled with oil, and a reaction solution 47 undergoes phase separation from the oil. Therefore, when being pushed out from the tube 20 to the PCR container 30, the reaction solution plug 47 becomes a droplet. The reaction solution 47 in the form of a droplet is precipitated since the specific gravity thereof is greater than that of the oil. When a high-temperature region 36A and a low-temperature region 36B are formed in the PCR container 30 by external heaters, and the entire cartridge 1 is repeatedly moved upside down together with the heater, the reaction solution 47 in the form of a droplet alternately moves between the high-temperature region 36A and the low-temperature region 36B, whereby two-stage temperature process is performed on the reaction solution 47 as a PCR solution.

The material of the PCR container 30 is not particularly limited, and the PCR container 30 can be formed of, for example, glass, resins such as plastic, and metals. Furthermore, it is preferable for the material of the PCR container 30 to be heat resistant to a temperature of at least 100°C or higher since the high temperature-side heater 65B is disposed near the container. It is preferable to select transparent or semitransparent materials as the material of the PCR container 30, since this makes it easy to perform fluorescence assay (luminance measurement). However, the entire region of the PCR container 30 does not need to be transparent or semitransparent, and at least the site (for example, a bottom 35A of the PCR container 30) facing a fluorescence measuring instrument 55 may be transparent or semitransparent. In addition, the PCR container 30 and the tank 3 or the plunger 10 may be made of the same material.

The cartridge 1 is constituted with the tank 3 and a cartridge main body 9. In a kit constituting the cartridge 1, the tank 3, and cartridge main body 9, and an adapter 5 are prepared in advance. The tank 3 and the cartridge main body 9 are connected to each other through the adapter 5, whereby the cartridge 1 is assembled. However, the cartridge 1 can also be constituted by directly connecting the tank 3 to the cartridge main body 9.

In the following description of the constituents of the cartridge 1, as described in Fig. 2A, the direction extending along the long cartridge 1 is described as a "longitudinal direction", the side of the tank 3 is described as an "upstream side", and the side of the PCR container 30 is described as a "downstream side". Moreover, in some cases, the upstream side and the downstream side are simply described as "upper side" and "lower side" respectively.

### 1. Tank

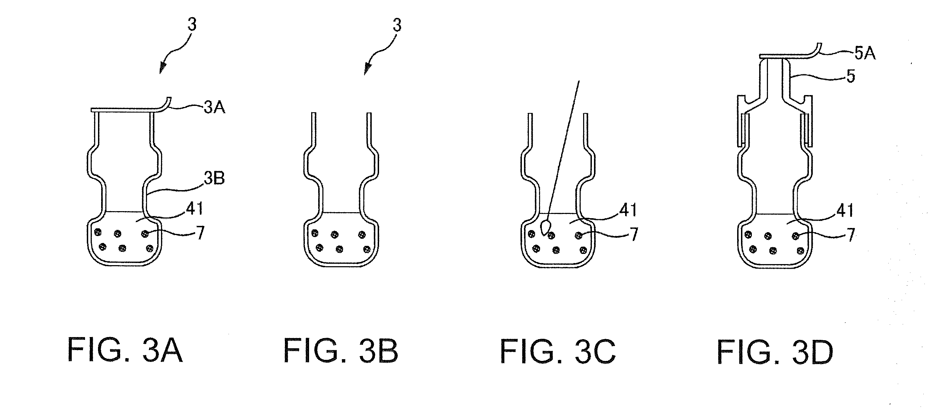

Figs. 3A to 3D are views for illustrating the tank 3.

The tank 3 prepared in advance in the kit contains a lysing solution 41 and magnetic beads 7. The opening of the tank 3 is provided with a detachable lid 3A (see Fig. 3A). As the lysing solution 41, 5 M guanidine thiocyanate, 2% Triton X-100, and 50 mM Tris-HCl (pH 7.2) are used. An operator removes the lid 3A to open the opening of the tank 3 (see Fig. 3B), and dips a cotton swab smeared with a virus into the lysing solution 41 in the tank 3 to collect the virus in the lysing solution 41 (see Fig. 3C). When the liquid in the tank 3 is stirred, the tank 3 may be shaken in the state described in Fig. 3C. However, in doing so, the lysing solution 41 easily overflows. Accordingly, it is preferable to shake the tank 3 after the adapter 5 covered with a lid 5A is mounted on the tank 3 as shown in Fig. 3D. In this manner, the substance in the tank 3 is stirred, the virus particles undergoes lysis by the lysing solution 41, whereby the nucleic acid is liberated, and silica that has coated the magnetic beads 7 absorbs the nucleic acid. The magnetic beads 7 correspond to the nucleic acid-binding solid-phase carriers. Thereafter, the operator detaches the lid 5a of the adapter 5 mounted on the opening of the tank 3, and mounts the tank 3 on the cartridge main body 9 through the adapter 5 (see Fig. 2A).

The tank 3 is constituted with a flexible resin and is dilatable. When the state of the cartridge changes as shown in Fig. 2B from the state shown in Fig. 2A due to sliding of the plunger 10, the tank 3 dilates, and this prevents the pressure of the liquid in the tube 20 from excessively increasing and from being pushed out to the downstream side. It is desirable to form a deformation portion 3B in the tank 3 such that the tank 3 easily dilates.

The sample used for the extraction and amplification of nucleic acid is not limited to viruses and may be cells. The sources of cells are not particularly limited and may be microorganisms, tissue fragments or blood of higher organisms, and the like.

The lysing solution 41 is not particularly limited as long as it contains a chaotropic substance, and may contain a surfactant to destruct the cell membrane or denature proteins contained in the cells. The surfactant is not particularly limited as long as it is generally used for extracting nucleic acid from cells and the like. Specific examples thereof include nonionic surfactants including triton-based surfactants such as Triton-X or tween-based surfactants such as Tween-20, and anionic surfactants such as N-lauroylsarcosine sodium (SDS). However, it is particularly preferable to use nonionic surfactants at a concentration within a range of 0.1% to 2%. Moreover, it is preferable for the lysing solution to contain a reductant such as 2-mercaptoethanol or dithiothreitol. The lysing solution may be a buffer solution, and pH thereof is preferably neutral such as pH 6 to 8. Considering the above, specifically, it is preferable for the lysing solution to contain 3 M to 7 M of a guanidine salt, 0% to 5% of a nonionic surfactant, 0 mM to 0.2 mM of EDTA, 0 M to 0.2 M of a reductant, and the like.

The chaotropic substance acts to generate chaotropic ions (monovalent anions having a large diameter) in an aqueous solution and enhance water solubility of hydrophobic molecules. This substance is not particularly limited as long as it contributes to the adsorption of nucleic acid onto the solid-phase carriers. Specific examples thereof include guanidine thiocynate, guanidine hydrochloride, sodium iodide, potassium iodide, sodium perchlorate, and the like. Among these, guanidine thiocynate or guanidine hydrochloride having a potent protein denaturating action is preferable. The concentration of these chaotropic substances used varies with the type of each of the substances. For example, when guanidine thiocyanate is used, this substance is preferably used in a range of 3 M to 5.5 M, and when guanidine hydrochloride is used, this substance is preferably used at a concentration of 5 M or more.

The tool for collecting the sample is not particularly limited, and instead of a cotton swab, a spatula, a rod, a scraper, and the like may be selected according to the purpose.

The internal volume of the tank 3 is not particularly limited, and may be, for example, from 0.1 mL to 100 mL. The material of the tank 3 is not particularly limited, and the tank 3 can be made of, for example, glass, resins such as plastic, and metals. Particularly, it is preferable to select transparent glass or resins as the material of the tank, since the inside of the tank 3 can be observed from the outside. The tank 3 and each tube 20 may be formed by monolithic molding or may be detachable from each other. If flexible materials such as rubber, elastomers, and polymers are used as the material of the tank 3, it is possible to increase the internal pressure of the tank 3 by deforming the tank 3 in a state where the tank 3 is covered with a lid. As a result, it is possible to push the content of the tube 20 out of the tip of the tube toward the outside from the inside of the tube.

### 2. Cartridge main body

The cartridge main body 9 has the plunger 10, the tube 20, and the PCR container 30.

### 2-1. Plunger

Hereinafter, the plunger 10 will be described with reference to Figs. 2A to 2C.

The plunger 10 is a movable plunger pushing out liquid from the downstream side of the tube 20 which functions as a syringe. The plunger 10 functions to push out a predetermined amount of liquid in the tube 20 to the PCR container 30 from the end of the tube 20. The plunger 10 also functions to mount the tank 3 on the cartridge through the adapter 5.

The plunger 10 has a cylindrical portion 11 and a rod-like portion 12. The cylindrical portion 11 is disposed at tank 3 side (upstream side), and the rod-like portion 12 is disposed at the tube 20 side (downstream side). The rod-like portion 12 is supported by two plate-like ribs 13 from the inner wall of the downstream side of the cylindrical portion 11. The downstream side of the rod-like portion 12 protrudes toward the downstream side from the cylindrical portion 11.

The cylindrical portion 11 is opened toward the upstream side and the downstream side, and the inner wall of the cylindrical portion 11 functions as a path of liquid. The opening of the upstream side (tank 3 side) of the cylindrical portion 11 fits to the adapter 5. In the plunger 10 of the cartridge main body 9 which is prepared in advance as a kit, a detachable lid may be mounted on the opening of the upstream side of the cylindrical portion 11. The opening of the downstream side of the cylindrical portion 11 is positioned in the inside of the upper syringe 21 of the tube 20. The magnetic beads 7 introduced from the opening of the upstream side of the cylindrical portion 11, pass through the inside of the cylindrical portion 11, escape from of the inside and outside of the ribs 13, come out of the opening of the downstream side of the cylindrical portion 11, and introduced into the upper syringe 21 of the tube 20.

The downstream side of the cylindrical portion 11 fits to the inner wall of the upper syringe 21 of the tube 20. The cylindrical portion 11 can slide relative to the upper syringe 21 in the longitudinal direction while being coming into contact with the inside of the upper syringe 21 of the tube 20.

In the periphery of the opening of the upstream side of the cylindrical portion 11, a mounting board 11A for mounting the adapter 5 is formed. The mounting board 11A also functions as a portion which is pushed when the plunger 10 is pushed. When the mounting board 11A is pushed, the plunger 10 slides relative to the tube 20, whereby the state of the cartridge changes as described in Fig. 2C from the state described in Fig. 2A. When the plunger 10 moves to the downstream side, the mounting board 11A comes into contact with the upper rim of the tube 20 (see Fig. 2C). That is, the interval between the mounting board 11A of the plunger 10 and the upper rim of the tube 20 is a sliding length of the plunger 10.

In the initial state, the rod-like portion 12 is positioned in the inside of the upper syringe 21 of the tube 20 and is separated from the lower syringe 22 (see Fig. 2A). When the plunger 10 slides relative to the tube 20, the rod-like portion 12 is inserted into the lower syringe 22 of the tube 20 and slides relative to the lower syringe 22 in the downstream direction while coming into contact with the inside of the lower syringe 22 (see Figs. 2B and 2C).

The cross-section of the rod-like portion 12 that is perpendicular to the longitudinal direction has a circular shape. However, the cross-sectional shape of the rod-like portion 12 may be circular, oval, or polygonal and is not particularly limited, as long as the rod-like portion 12 can fit to the inner wall of the lower syringe 22 of the tube 20.

The seal 12A is formed at the edge of the downstream side of the rod-like portion 12. When the seal 12A fits into the lower syringe 22, the liquid in the tube 20 of the downstream side is prevented from flowing back toward the upper syringe 21. Moreover, while the plunger 10 is being pushed such that the state of the cartridge changes to the state described in Fig. 2C from the state described in Fig. 2B, the liquid in the tube 20 is pushed out of the downstream side, by the amount corresponding to the volume in which the seal 12A slides inside the lower syringe 22.

The volume in which the seal 12A slides inside the lower syringe 22 (the amount of the liquid in the tube 20 that is pushed out from the downstream side) is larger than the total volume of the reaction solution plug 47 and a third oil plug 48 in the tube 20. Consequently, the liquid in the tube 20 can be pushed out such that the reaction solution 47 does not remain in the tube 20.

The material of the plunger 10 is not particularly limited, and the plunger 10 can be made of, for example, glass, resins such as plastic, and metals. Moreover, the cylindrical portion 11 and the rod-like portion 12 of the plunger 10 may be formed of the same material as a monolithic member, or may be formed of different materials. Herein, the cylindrical portion 11 and the rod-like portion 12 are separately molded with a resin, and the cylindrical portion 11 is joined with the rod-like portion 12 through the ribs 13, whereby the plunger 10 is formed.

The plunger 10 accommodates in advance oil 42 and a first washing solution 43 in the inside thereof. The specific gravity of the oil 42 in the plunger 10 is smaller than that of the first washing solution 43. Accordingly, when the tank 3 is mounted on the cartridge main body 9, if the cartridge main body 9 is made to stand such that the mounting board 11A of the plunger 10 becomes the upper side, the oil 42 is disposed between the liquid in the tank 3 and the first washing solution 43 of the cartridge main body 9 as shown in Fig. 2A. As the oil 42, 2CS silicone oil is used, and as the first washing solution 43, 8 M guanidine hydrochloride and 0.7% Triton X-100 are used.

The first washing solution 43 may be liquid that undergoes phase separation when being mixed with any of the oil 42 and oil 44. It is preferable for the first washing solution 43 to be water or an aqueous solution with a low salt concentration, and the aqueous solution with a low salt concentration is preferably a buffer solution. The salt concentration of the aqueous solution with a low salt concentration is preferably 100 mM or less, more preferably 50 mM or less, and most preferably 10 mM or less. The lower limit of the salt concentration of the aqueous solution with a low salt concentration is not particularly limited, but is preferably 0.1 mM or higher, more preferably 0.5 mM or higher, and most preferably 1 mM or higher. This solution may also contain a surfactant such as Triton, Tween, or SDS, and pH thereof is not particularly limited. The type of salt for making the buffer solution is not particularly limited, and salts of TRIS, HEPES, PIPES, phosphoric acid, and the like are preferably used. In addition, it is preferable for this washing solution to contain alcohol in such an amount that does not hinder the adsorption of nucleic acid onto carriers, a reverse transcription reaction, a PCR reaction, and the like. In this case, the concentration of alcohol is not particularly limited and may be 70% or less, 60% or less, 50% or less, 40% or less, 30% or less, 20% or less, or 10% or less. However, the concentration is preferably 5% or less or 2% or less, more preferably 1% or less or 0.5% or less, and most preferably 0.2% or less or 0.1% or less.

The first washing solution 43 may contain a chaotropic agent. For example, if the first washing solution 43 contains guanidine hydrochloride, it is possible to wash particles and the like while maintaining or reinforcing the adsorption state of nucleic acid having been adsorbed onto the particles and the like. When the washing solution contains guanidine hydrochloride, the concentration of this agent can be controlled to be 3 mol/L to 10 mol/L and preferably from 5 mol/L to 8 mol/L. If the concentration of guanidine hydrochloride is within the above range, it is possible to wash off foreign substances and the like while further stabilizing the adsorption state of nucleic acid having been adsorbed onto particles and the like.

### 2-2. Tube

Hereinafter, the tube 20 will be described with reference to Figs. 2A to 2C.

The tube 20 has the shape of a cylinder through which liquid can flow in the longitudinal direction. The tube 20 has the upper syringe 21, the lower syringe 22, and a capillary 23, and the inner diameter of each of these portions varies stepwise.

The upper syringe 21 has the shape of a cylinder through which liquid can flow in the longitudinal direction. The cylindrical portion 11 of the plunger 10 slidably comes into contact with the inside of the inner wall of the upper syringe 21. The upper syringe 21 functions as a syringe for the cylindrical portion 11 of the plunger 10.

The lower syringe 22 has the shape of a cylinder through which liquid can flow in the longitudinal direction. The seal 12A of the rod-like portion 12 of the plunger 10 can slidably fit to the inner wall of the lower syringe 22. The lower syringe 22 functions as a syringe for the rod-like portion 12 of the plunger 10.

The capillary 23 has the shape of a capillary tube through which liquid can flow in the longitudinal direction. The inner diameter of the capillary 23 has such a size that enables the liquid to be maintained in the form of a plug. Herein, the inner diameter is 1.0 mm. The inner diameter of terminal (terminal of the downstream side of the tube 20) of the capillary 23 is 0.5 mm which is smaller than the aforementioned diameter. The inner diameter of terminal of the capillary 23 is set to be smaller than the diameter (1.5 mm to 2.0 mm) of the reaction solution, which will be described later, in the form of a droplet. Accordingly, when the reaction solution plug 47 is pushed out of the terminal of the capillary 23, the reaction solution in the form of a droplet can be inhibited from adhering to the terminal of the capillary 23 or flowing back into the capillary 23.

The capillary 23 may have a cavity in the inside thereof and have the shape of a cylinder through which liquid can flow in the longitudinal direction. The capillary 23 may be curved in the longitudinal direction, but it is preferable for the capillary 23 to be straight. The internal cavity of the tube is not particularly limited in terms of the size and shape, as long as liquid can be maintained in the form of a plug inside the tube. Moreover, the size of the internal cavity of the tube or the shape of a cross-section thereof perpendicular to the longitudinal direction may be varied along the longitudinal direction of the tube.

The shape of the cross-section of the tube that is perpendicular to the longitudinal direction of the exterior of the tube is not limited. Moreover, the thickness (a distance between the lateral wall of the internal cavity and the exterior surface) of the tube is not particularly limited. When the tube has the shape of a cylinder, the inner diameter (diameter of a circle which is a cross-section perpendicular to the longitudinal direction of the internal cavity) can be set to, for example, 0.5 mm to 2 mm. If the inner diameter of the tube is within the above range, it is easy to form a plug of liquid within a wide range of tube materials and liquid type. It is preferable for the tube to be tapered toward the tip, and the diameter of the tip can be set to 0.2 mm to 1 mm. If the inner diameter of the terminal of the capillary 23 (diameter of opening of the capillary 23) is set to be small, it is possible to inhibit the reaction solution 47, which has been made into a droplet in the PCR container 30, from being adsorbed onto the opening of the capillary 23 and becoming inseparable. However, if the inner diameter of terminal of the capillary 23 is too small, a large number of small droplets of the reaction solution 47 are formed. In addition, in the capillary 23, if the diameter of portion other than the terminal is made small just like the terminal, it is not desirable since the cartridge 1 will be lengthened to secure the volume of each plug.

In the inside of the capillary 23, a first oil plug 44, a washing solution plug 45, a second oil plug 46, the reaction solution plug 47, and a third oil plug 48 are present in this order from the upstream side. That is, oil plugs are disposed at both sides of a water-soluble plug (the washing solution plug 45 or the reaction solution plug 47).

In the upper syringe 21 and the lower syringe 22 at the upstream side from the first oil plug 44, the oil 42 and the washing solution 43 have already been accommodated (see Fig. 2A). The inner diameter of the upper syringe 21 and the lower syringe 22 is larger than the inner diameter of the capillary 23. In the upper syringe 21 and the lower syringe 22, the liquid (the oil 42 and the washing solution 43) cannot be maintained in the form of a column just like a plug. However, since the first oil plug 44 is held in the form of a plug by the capillary 23, the oil constituting the first oil plug 44 is inhibited from moving to the upstream side.

The washing solution plug 45 may be formed of 5 mM of tris-hydrochloric acid buffer solution which is a second washing solution. The second washing solution may be constituted basically in the same manner as the first washing solution described above or may be the same as or different from the first washing solution. However, in order to prevent a chaotropic substance from being mixed into the solution after the second washing solution, it is preferable for the second washing solution to be a solution which substantially does not contain the chaotropic substance. As described above, it is preferable for this washing solution to contain alcohol in such an amount that does not hinder the adsorption of nucleic acid onto carriers, a reverse transcription reaction, a PCR reaction, and the like. In this case, the concentration of alcohol is not particularly limited and may be 70% or less, 60% or less, 50% or less, 40% or less, 30% or less, 20% or less, or 10% or less. However, the concentration is preferably 5% or less or 2% or less, more preferably 1% or less or 0.5% or less, and most preferably 0.2% or less or 0.1% or less.

The washing solution plug 45 may be constituted with plural plugs divided by oil plugs. When the washing solution plug 45 consists of plural plugs, the liquids of the respective plugs may be the same as or different from one another. If at least one of the plugs is a plug of a washing solution, the liquids of other plugs are not particularly limited. However, it is preferable for all plugs to be washing solutions. The number of divided plugs of the washing solution plug 45 can be appropriately set in consideration of, for example, the length of the tube 20 or the subject to be washed.

The reaction solution plug 47 is formed of, for example, the following reaction solution.

| | |
|---|---|
| 0.2 u/µL | AMV reverse transcriptase (manufactured by NIPPON GENE CO., LTD.) |
| 0.125 u/µL | Gene Taq NT PCR polymerase (manufactured by NIPPON GENE CO., LTD.) |
| 0.5 mM | dNTP |
| 1.0 µM | primer (forward) |
| 1.0 µM | primer (reverse) |
| 0.5 µM | probe (Taq man) |
| 4.0 mg/mL | BSA |
| x1 | buffer (7 mM MgCl₂; 0.25 mM Tris with pH 9.0,; 50 mM KCl) |

The reaction solution 47 refers to the liquid that causes the nucleic acid having been adsorbed onto the nucleic acid-binding solid-phase carriers to be eluted in the solution to cause a reverse transcription reaction and a polymerase reaction. Therefore, the reaction solution 47 is adjusted in advance such that the reaction solution 47, in which the nucleic acid has been eluted, is directly utilized as a buffer solution used for a reverse transcription reaction or a polymerase reaction. The reaction solution 47 contains an eluate that causes the elution of the nucleic acid.

For a reverse transcription reaction, the reaction solution 47 contains a reverse transcriptase, dNTP, and a primer for a reverse transcriptase (oligonucleotide). Moreover, for a polymerase reaction, the reaction solution 47 contains a DNA polymerase and a primer (oligonucleotide) for a DNA polymerase and may contain a TaqMan probe, a probe for real time PCR, such as Molecular Beacon or a cycling probe, or a fluorescent dye for an intercalator such as SYBR green. Moreover, it is preferable for the reaction solution 47 to contain bovine serum albumin (BSA) or gelatin as a reaction hindrance inhibitor. The solvent is preferably water and more preferably a solvent that substantially does not contain an organic solvent such as ethanol or isopropanol and a chaotropic substance. Furthermore, it is preferable for the reaction solution 47 to contain a salt such that the reaction solution becomes a buffer solution for a reverse transcriptase and/or a buffer solution for a DNA polymerase. The salt that makes the reaction solution be a buffer solution is not particularly limited as long as the salt does not hinder an enzyme reaction. However, salts of TIS, HEPES, PIPES, phosphoric acid, and the like are preferably used. The reverse transcriptase is not particularly limited, and for example, it is possible to use reverse transcriptase derived from avian myeloblast virus, ras-associated virus type 2, mouse moloney murine leukemia virus, and human immunodeficiency virus type 1. However, it is preferable to use heat-resistant enzymes. The DNA polymerase is not particularly limited, but it is preferable to use heat-resistant enzymes or enzymes for PCR. For example, there are an extremely large number of commercially available products such as Taq polymerase, Tfi polymerase, Tth polymerase, and enzymes obtained by modifying the above enzymes. However, it is preferable to use DNA polymerase that can be subjected to hot start.

The sequence of the primer for DNA polymerase can be easily and appropriately determined for the DNA to be detected. Usually, in order to amplify one kind of DNA, the reaction solution may contain a pair of primers including a 5' primer and a 3' primer. Moreover, if plural kinds of primer pairs marked with different types of fluorescent dyes are added in advance to the reaction solution to amplify plural kinds of DNA, the reaction solution can be used for multiplex PCR. In this case, the number of TaqMan probe may also be appropriately increased to be plural.

The concentration of dNTP or salt contained in the reaction solution may be set appropriately depending on the type of the enzyme to be used. However, generally, the concentration of dNTP is 10 µM to 1,000 µM and preferably 100 µM to 500 µM, the concentration of Mg2+ is preferably 1 mM to 100 mM and preferably 5 mM to 10 mM, and the concentration of Cl- is 1 mM to 2,000 mM and preferably 200 mM to 700 mM. The total ion concentration is not particularly limited, but is preferably higher than 50 mM, more preferably higher than 100 mM, even more preferably higher than 120 mM, still more preferably higher than 150 mM, and yet more preferably higher than 200 mM. The upper limit thereof is preferably 500 mM or less, more preferably 300 mM or less, and even more preferably 200 mM or less. The oligonucleotides for a primer are used at a concentration of 0.1 µM to 10 µM, and preferably at a concentration of 0.1 µM to 1 µM respectively. If the concentration of BSA or gelatin is 1 mg/mL or less, the reaction hindrance inhibitory effect is diminished, and if the concentration is 10 mg/mL or higher, the reverse transcription reaction and the following enzyme reaction may be hindered. Accordingly, the concentration is preferably 1 mg/mL to 10 mg/mL. When gelatin is used, the source thereof is not particularly limited, and examples thereof include cowhide, pig hide, and beef bones. When the gelatin does not easily dissolve, it may be dissolved by heating.

The volume of the reaction solution plug 47 is not particularly limited and can be appropriately set based on an index such as the amount of particles and the like onto which nucleic acid has been adsorbed. For example, when the volume of the particles and the like is 0.5 µL, a volume of 0.5 µL or more is sufficient as the volume of the reaction solution plug 47. The volume is preferably from 0.8 µL to 5 µL, and more preferably from 1 µL to 3 µL. If the volume of the reaction solution plug 47 is within the above range, it is possible to cause the nucleic acid to be sufficiently eluted from the carriers even if the volume of the nucleic acid-binding solid-phase carriers is set to 0.5 µL.

The downstream portion of the capillary 23 is inserted into the PCR container 30. As a result, by pushing the reaction solution plug 47 in the tube 20 out of the tube 20, the reaction solution 47 can be introduced into the PCR container 30.

A cyclic convexity of the outer wall of the capillary 23 comes into contact with the inner wall of the PCR container 30, whereby an upper seal portion is formed. Furthermore, the outer wall of the capillary 23 at the downstream side from the upper seal portion comes into contact with the inner wall of the PCR container 30, whereby a lower seal portion is formed. The upper seal portion and lower seal portion will be described later.

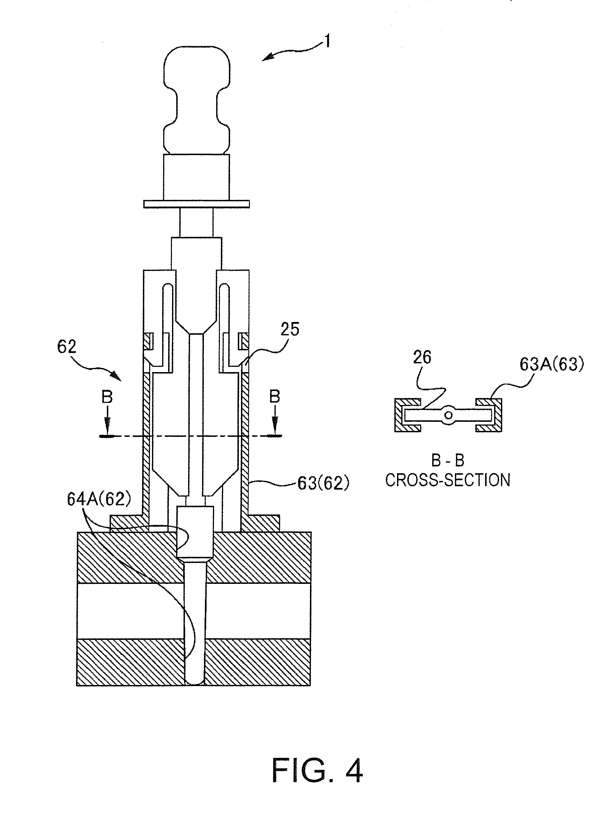

The tube 20 further has the fixing claw 25 and the guide plate 26. Fig 4 is a view for illustrating the fixing claw 25, the guide plate 26, and the mounting portion 62.

The fixing claw 25 is a member for fixing the cartridge 1 to the mounting portion 62. When the cartridge 1 is inserted into the mounting portion 62 until the fixing claw 25 is hooked on, the cartridge 1 is fixed to a normal position of the mounting portion 62. In other words, when the cartridge 1 is in an abnormal position of the mounting portion 62, the fixing claw 25 is not hooked on the mounting portion 62.

The guide plate 26 is a member for guiding the cartridge 1 when the cartridge 1 is mounted on the mounting portion 62 of the PCR device 50. A guide rail 63A is formed in the mounting portion 62 of the PCR device 50. The cartridge 1 is inserted into and fixed to the mounting portion 62 while being guided along the guide rail 63A by the guide plate 26 of the tube 20. The cartridge 1 is long. However, since the cartridge 1 is inserted into the mounting portion 62 under the guidance of the guide plate 26, it is easy to fix the cartridge 1 to a normal position of the mounting portion 62.

The fixing claw 25 and the guide plate 26 are plate-like members protruding from the right and left of the capillary 23. When the magnetic beads 7 in the tube 20 are moved by using a magnet, the magnet is caused to approach the tube, in the direction perpendicular to the plate-like fixing claw 25 or guide plate 26. Accordingly, the distance between the magnet and the magnetic beads 7 in the tube 20 can be shortened. However, for shortening the distance between the magnet and the magnetic beads 7 in the tube 20, the fixing claw 25 and the guide plate 26 may have other shapes.

### 2-3. PCR container

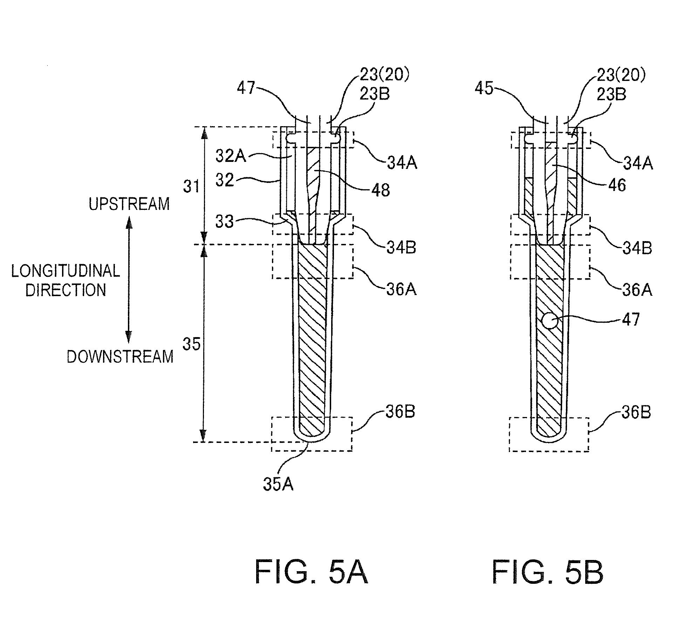

Figs. 5A and 5B are views for illustrating the periphery of the PCR container 30. Fig. 5A is a view for illustrating the initial state. Fig 5B is a view for illustrating the state where the plunger 10 has been pushed. Hereinafter, Figs. 2A to 2C will also be referred to for describing the PCR container 30.

The PCR container 30 is a container which receives the liquid pushed out of the tube 20 and accommodates the reaction solution 47 during the thermal cycling process. The PCR container corresponds to a nucleic acid amplification reaction container.

The PCR container 30 has a seal formation portion 31 and a flow path formation portion 35. The seal formation portion 31 is a portion in which the tube 20 has been inserted and which inhibits the oil, which overflows from the flow path formation portion 35, from leaking to the outside. The flow path formation portion 35 is placed at the downstream side from the seal formation portion 31 and forms a flow path through which the reaction solution 47 in the form of a droplet moves. The PCR container 30 is fixed to the tube 20 at two sites including an upper seal portion 34A and a lower seal portion 34B of the seal formation portion 31.

The seal formation portion 31 has an oil accommodating portion 32 and a stepped portion 33.

The oil accommodating portion 32 is a cylindrical portion and functions as a reservoir accommodating the oil that overflows from the flow path formation portion 35. There is a gap between the inner wall of the oil accommodating portion 32 and the outer wall of the capillary 23 of the tube 20, and this gap becomes an oil accommodating space 32A accommodating the oil that overflows from the flow path formation portion 35. The volume of the oil accommodating space 32A is larger than the volume in which the seal 12A of the plunger 10 slides in the lower syringe 22 of the tube 20.

The inner wall of the upstream side of the oil accommodating portion 32 comes into contact with the cyclic convexity of the tube 20, whereby the upper seal portion 34A is formed. The upper seal portion 34A is a seal that allows permeation of air while inhibiting the oil of the oil accommodating space 32A from leaking to the outside. In the upper seal portion 34A, a vent having such a size that does not allow the oil to leak due to the surface tension of the oil is formed. The vent of the upper seal portion 34A may be a gap between the convexity of the tube 20 and the inner wall of the oil accommodating portion 32, or may be a hole, groove, or notch formed in the convexity of the tube 20. Moreover, the upper seal portion 34A may be formed of an oil absorbent absorbing oil.

The stepped portion 33 is a portion which is disposed at the downstream side of the oil accommodating portion 32 and shows a step difference. The inner diameter of the downstream portion of the stepped portion 33 is smaller than the inner diameter of the oil accommodating portion 32. The inner wall of the stepped portion 33 comes into contact with the outer wall of the downstream side of the capillary 23 of the tube 20. The inner wall of the stepped portion 33 comes into contact with the outer wall of the tube 20, whereby the lower seal portion 34B is formed. The lower seal portion 34B is a seal that allows the oil of the flow path formation portion 35 to flow to the oil accommodating space 32A while exhibiting resistance to the flow. Due to pressure loss of the lower seal portion 34B, the pressure in the flow path formation portion 35 becomes higher than the outside pressure. Therefore, even if the liquid in the flow path formation portion 35 is heated during the thermal cycling process, air bubbles are not easily formed in the liquid in the flow path formation portion 35.

The flow path formation portion 35 is a tubular portion and functions as a container forming a flow path through which the reaction solution 47 in the form of a droplet moves. The flow path formation portion 35 is filled with oil. The upstream side of the flow path formation portion 35 is closed by the terminal of the tube 20, and the terminal of the tube 20 is opened toward the flow path formation portion 35. The inner diameter of the flow path formation portion 35 is larger than the inner diameter of the capillary 23 of the tube 20, and is also larger than the outer diameter of a sphere of the liquid having the volume of the reaction solution plug 47 that has been made into a sphere. It is desirable for the inner wall of the flow path formation portion 35 to exhibit water repellency to such a degree that the water-soluble reaction solution 47 does not adhere to the inner wall.

The upstream side of the flow path formation portion 35 is heated at a relatively high temperature (for example, about 95°C) by the high temperature-side heater 65B of the outside, thereby forming a high-temperature region 36A. The downstream side of the flow path formation portion 35 is heated at a relatively low temperature (for example, about 60°C) by the low temperature-side heater 65C of the outside, thereby forming a low-temperature region 36B. The bottom 35A (edge of the downstream side) of the PCR container 30 includes the low-temperature region 36B. As a result, a temperature gradient is formed in the liquid in the flow path formation portion 35.

As shown in Fig. 5A, in the initial state, oil is filled in the flow path formation portion 35 of the PCR container 30. The interface of the oil is positioned at the relatively downstream side from the oil accommodating space 32A. In the oil accommodating space 32A, the volume of the upstream side from the interface of the oil is larger than the volume in which the seal 12A of the plunger 10 slides in the lower syringe 22 of the tube 20.

As shown in Fig. 5B, when the plunger 10 is pushed, the liquid in the tube 20 is pushed out into the flow path formation portion 35. Since the liquid in the tube 20 is pushed out into the flow path formation portion 35 which has already been filled with oil, gas does not flow into the flow path formation portion 35.

When the plunger 10 is pushed, first, the third oil plug 48 of the tube 20 flows into the flow path formation portion 35, and the inflow oil then flows into the oil accommodating space 32A from the flow path formation portion 35, whereby the oil interface of the oil accommodating space 32A ascends. At this time, due to pressure loss of the lower seal portion 34B, the pressure of liquid in the flow path formation portion 35 increases. After the third oil plug 48 is pushed out of the tube 20, the reaction solution plug 47 flows into the flow path formation portion 35 from the tube 20. Since the inner diameter of the flow path formation portion 35 is larger than the inner diameter of the capillary 23, the reaction solution 47, which has been in the form of a plug (form of a column) in the tube 20, becomes a droplet in the oil in the flow path formation portion 35. Moreover, in the initial state, the volume of the oil accommodating space 32A that is at the upstream side from the interface of oil is larger than the volume in which the seal 12A of the plunger 10 slides in the lower syringe 22 of the tube 20. Consequently, the oil does not overflow from the oil accommodating space 32A.

### PCR device 50

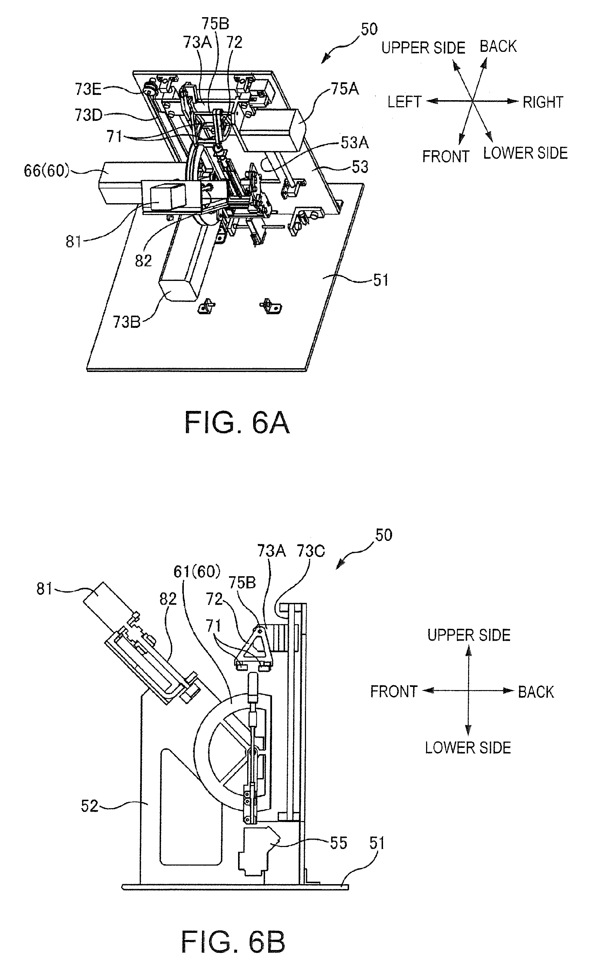

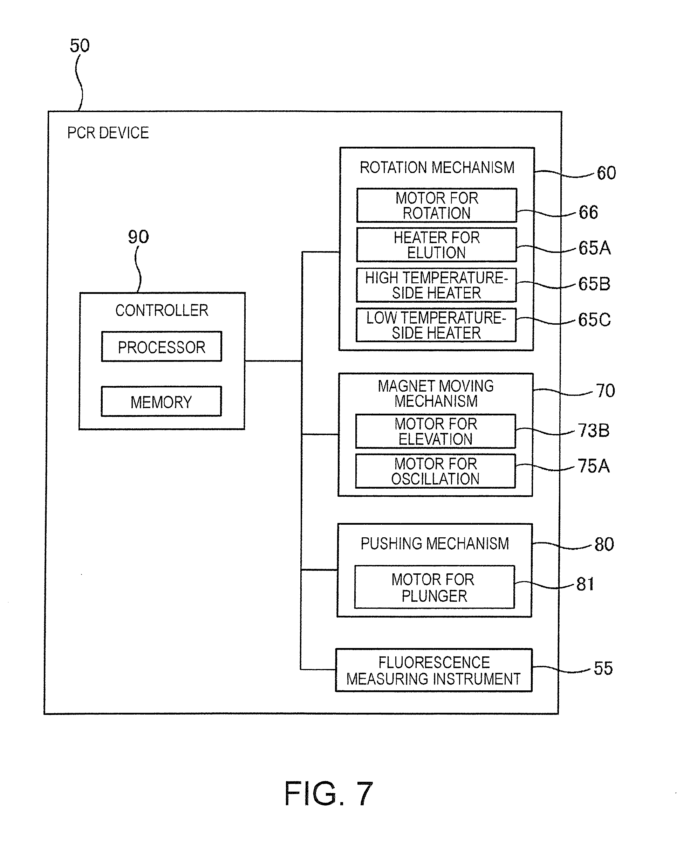

Fig. 6A is a perspective view showing the internal constitution of the PCR device 50. Fig 6B is a lateral view showing the main constitution of the PCR device 50. Fig. 7 is a block diagram of the PCR device 50. The PCR device 50 is a device performing nucleic acid elution process and thermal cycling process by using the cartridge 1.

Hereinafter, the meaning of "upper and lower", "front and back", and "left and right" will be defined as shown in the drawing to describe the PCR device 50. When a base 51 of the PCR device 50 is horizontally disposed, a direction perpendicular to the base 51 is defined as "vertical direction", and "upper" and "lower" will be defined according to the direction of gravity. Moreover, the axial direction of the rotary shaft of the cartridge 1 is defined as "horizontal direction", and a direction perpendicular to the vertical direction and the horizontal direction is defined as "front-back direction". When the PCR device 50 is viewed from the rotary shaft of the cartridge 1, the side of a cartridge insertion port 53A is defined as "back", and the side opposite to the "back" is defined as "front". When the PCR device 50 is viewed from the front side, the right side and left side in the horizontal direction are defined as "right" and "left" respectively.

The PCR device 50 has a rotation mechanism 60, a magnet moving mechanism 70, a pushing mechanism 80, the fluorescence measuring instrument 55, and a controller 90.

### 1. Rotation mechanism 60

The rotation mechanism 60 is a mechanism for rotating the cartridge 1 and the heater. When the cartridge 1 and the heater become upside down by the rotation mechanism 60, the reaction solution 47 in the form of a droplet moves inside the flow path formation portion 35 of the PCR container 30, whereby thermal cycling process is performed.

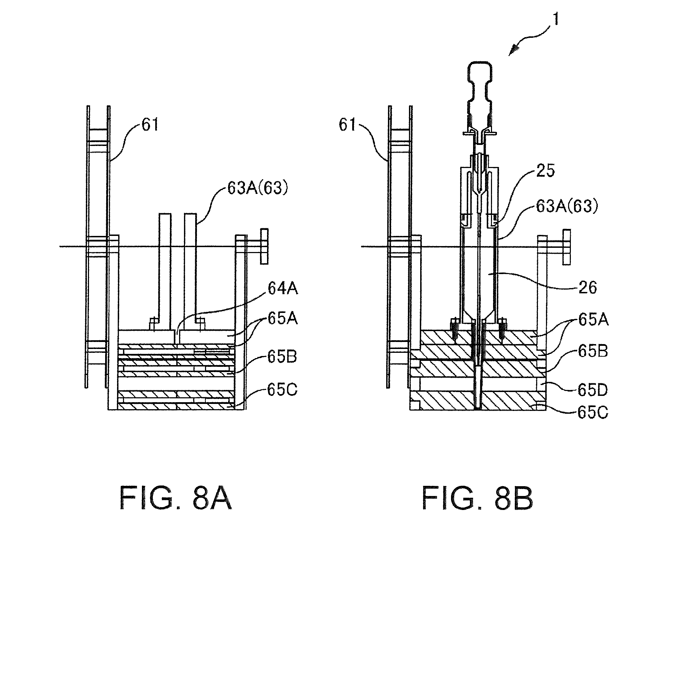

The rotation mechanism 60 has a rotary body 61 and a motor for rotation 66. Fig. 8A is a view for illustrating the rotary body 61. Fig. 8B is a view for illustrating the state where the cartridge 1 has been mounted on the mounting portion 62 of the rotary body 61.

The rotary body 61 is a member that can rotate around the rotary shaft. The rotary shaft of the rotary body 61 is supported on a support 52 fixed to the base 51. The rotary body 61 is provided with the mounting portion 62 onto which the cartridge 1 is mounted and the heaters (the heater for elution 65A, the high temperature-side heater 65B, and the low temperature-side heater 65C) . When the rotary body 61 rotates, the cartridge 1 can become upside down in a state where the positional relationship between the cartridge 1 and the heaters are maintained. The motor for rotation 66 is a source of power for rotating the rotary body 61. According to the instruction from the controller 90, the motor for rotation 66 causes the rotary body 61 to rotate to a predetermined position. A transmission mechanism such as a gear may be disposed between the motor for rotation 66 and the rotary body 61.

The rotary shaft of the rotary body 61 is positioned closer to the tube 20 than to the PCR container 30 of the cartridge 1. In other words, the rotary shaft of the rotary body 61 is positioned in the tube 20 of the cartridge 1 mounted on the mounting portion 62. This is because, since the tube 20 is longer than the PCR container 30, if the center of the PCR container 30 is used as the rotary shaft (if the rotary shaft of the rotary body 61 is positioned in the PCR container), the size of the rotary body 61 will increase.

The mounting portion 62 is a portion on which the cartridge 1 is mounted. The mounting portion 62 has a fixing portion 63 in which a notch is formed. Moreover, an insertion hole 64A formed in the heaters (the heater for elution 65A, the high temperature-side heater 65B, and the low temperature-side heater 65C) also functions as the mounting portion 62. When the fixing claw 25 of the cartridge 1 is hooked on the notch of the fixing portion 63 in a state where the PCR container 30 has been inserted into the insertion hole 64A, the cartridge 1 is mounted on the rotary body 61 (see Fig. 4) . Herein, a portion of the heater also functions as the mounting portion 62, but the mounting portion 62 may be separated from the heater. The mounting portion 62 is indirectly fixed to the rotary body 61 through the heater for elution 65A. However, the mounting portion 62 may be directly disposed in the rotary body 61. Moreover, the number of the cartridge 1 that can be mounted on the mounting portion 62 is not limited to one, and plural cartridges 1 may be mounted on the mounting portion 62.

The fixing portion 63 of the mounting portion 62 functions as a tube fixing portion to which the tube 20 of the cartridge 1 is fixed. The insertion hole 64A functions as a PCR container fixing portion to which the PCR container 30 is fixed. In this manner, the long cartridge 1 including the tube 20 and the PCR container 30 is stably fixed to the mounting portion 62.

The guide rail 63A is formed in the fixing portion 63 along the vertical direction (see Fig. 4). The guide rail 63A guides the guide plate 26 of the cartridge 1 in the insertion direction while restraining the guide plate 26 in the front-back direction. While the guide plate 26 is being guided by the guide rail 63A, the cartridge 1 is inserted into the mounting portion 62. Consequently, the PCR container 30 of the cartridge 1 is guided to the insertion hole 64A, whereby the cartridge 1 is fixed to a normal position of the mounting portion 62.

The PCR device 50 has the high temperature-side heater 65B and low temperature-side heater 65C, which are heaters for PCR, and the heater for elution 65A. Each of the heaters is constituted with heating source not shown in the drawing and a heat block. The heating source is, for example, a cartridge heater and has been inserted into the heat block. The heat block is, for example, a metal with high heat conductivity, such as aluminum, and heats the liquid in the cartridge 1 with the heat from the heating source while suppressing heat unevenness. It is desirable for the heat block to be a nonmagnetic substance to prevent the magnets 71, which moves the magnetic beads 7, from being adsorbed onto the heat block.

The heater for elution 65A is a heater that heats the reaction solution plug 47 of the cartridge 1. When the cartridge 1 is fixed to a normal position, the heater for elution 65A faces the reaction solution plug 47 of the tube 20. For example, the heater for elution 65A heats the reaction solution plug 47 to about 50°C, whereby liberation of nucleic acid from the magnetic beads is accelerated.

The high temperature-side heater 65B is a heater that heats the upstream side of the flow path formation portion 35 of the PCR container 30. When the cartridge 1 is fixed to a normal position, the high temperature-side heater 65B faces the upstream side (high-temperature region 36A) of the flow path formation portion 35 of the PCR container 30. For example, the high temperature-side heater 65B heats the liquid of the upstream side of the flow path formation portion 35 of the PCR container 30 to about 90°C to 100°C.

The low temperature-side heater 65C is a heater that heats the bottom 35A of the flow path formation portion 35 of the PCR container 30. When the cartridge 1 is fixed to a normal position, the low temperature-side heater 65C faces the downstream side (the low-temperature region 36B) of the flow path formation portion 35 of the PCR container 30. For example, the low temperature-side heater 65C heats the liquid in the low-temperature region 36B of the PCR container 30 to about 50°C to 75°C.

A spacer 65D is disposed between the high temperature-side heater 65B and the low temperature-side heater 65C. The spacer 65D suppresses heat conduction caused between the high temperature-side heater 65B and the low temperature-side heater 65C. The spacer 65D is also used for accurately setting the distance between the high temperature-side heater 65B and the low temperature-side heater 65C. In this manner, by the high temperature-side heater 65B and the low temperature-side heater 65C, a temperature gradient is formed in the liquid in the flow path formation portion 35 of the PCR container 30.

In each of the heat blocks constituting each of the heater for elution 65A, the high temperature-side heater 65B, and the low temperature-side heater 65C, a through hole constituting the insertion hole 64A is formed. The outer wall of the bottom 35A of the PCR container 30 is exposed through the opening of the lower side of the insertion hole 64A of the low temperature-side heater 65C. The fluorescence measuring instrument 55 measures the luminance of the reaction solution 47 from the opening of the lower side of the insertion hole 64A.

Each of the high temperature-side heater 65B and low temperature-side heater 65C is provided with a temperature control device. Accordingly, the temperature can be set to a level suitable for each polymerase reaction.

### 2. Magnet moving mechanism 70

The magnet moving mechanism 70 is a mechanism moving the magnets 71. The magnet moving mechanism 70 causes the magnetic beads 7 in the cartridge 1 to be attracted to the magnets 71 and moves the magnets 71 such that the magnetic beads 7 move to the inside of the cartridge 1. The magnet moving mechanism 70 has a pair of magnets 71, an elevating mechanism 73, and an oscillation mechanism 75.

The magnets 71 are members attracting the magnetic beads 7. As the magnets 71, a permanent magnet, an electromagnet, and the like can be used. However, herein, a permanent magnet that does not generate heat or the like is used. The pair of magnets 71 are held in an arm 72 while facing each other in the front-back direction, in a state where the position thereof in the vertical direction is almost the same. The respective magnets 71 can face each other at the front side or the back side of the cartridge 1 mounted on the mounting portion 62. The pair of magnets 71 can sandwich the cartridge 1 mounted on the mounting portion 62 therebetween in the front-back direction. If the magnets 71 are caused to face each other in a direction (herein, front-back direction) orthogonal to the direction (herein, horizontal direction) in which the fixing claw 25 or the guide plate 26 of the cartridge 1 is disposed, the distance between the magnetic beads 7 in the cartridge 1 and the magnets 71 can be shortened.

The elevating mechanism 73 is a mechanism for moving the magnets 71 in the vertical direction. Since the magnets 71 attract the magnetic beads 7, if the magnets 71 are moved in the vertical direction in accordance with the movement of the magnetic beads 7, the magnetic beads 7 in the cartridge 1 can be attracted in the vertical direction.

The elevating mechanism 73 has a carriage 73A moving in the vertical direction and a motor for elevation 73B. The carriage 73A is a member movable in the vertical direction. By a carriage guide 73C disposed in a lateral wall 53 where the cartridge insertion port 53A is placed, the carriage 73A is guided so as to be movable in the vertical direction. The arm 72 holding the pair of magnets 71 is mounted on the carriage 73A. Accordingly, when the carriage 73A moves in the vertical direction, the magnets 71 also move in the vertical direction. The motor for elevation 73B is a source of power for moving the carriage 73A in the vertical direction. According to the instruction from the controller 90, the motor for elevation 73B moves the carriage 73A to a predetermined position in the vertical direction. The motor for elevation 73B moves the carriage 73A in the vertical direction by using a belt 73D and a pulley 73E. However, the motor for elevation 73B may move the carriage 73A in the vertical direction by using other transmission mechanisms.

When the carriage 73A is in the uppermost position (retraction position), the magnets 71 are positioned above the cartridge 1. When the carriage 73A is in the retraction position, the elevating mechanism 73 does not come into contact with the cartridge 1 even if the cartridge 1 rotates. The elevating mechanism 73 can bring down the carriage 73A to a position where the magnets 71 face the reaction plug. Therefore, the elevating mechanism 73 can move the magnets 71 such that the magnetic beads 7 in the tank 3 move to the position of the reaction plug.

The oscillation mechanism 75 is a mechanism for causing the pair of magnets 71 to oscillate in the front-back direction. When the pair of magnets 71 is caused to oscillate in the front-back direction, each of the magnets 71 becomes distant from the cartridge 1 by different length. Since the magnetic beads 7 are attracted to the magnets 71 close to the cartridge 1, if the pair of magnets 71 is caused to oscillate in the front-back direction, the magnetic beads 7 in the cartridge 1 move in the front-back direction.

The oscillation mechanism 75 has a motor for oscillation 75A and a gear. The motor for oscillation 75A and the gear are disposed in the carriage 73A and can move in the vertical direction together with the carriage 73A. When the power of the motor for oscillation 75A is transmitted to the arm 72 through the gear, the arm 72 holding the magnets 71 rotates around an oscillating rotary shaft 75B relative to the carriage 73A. In order to prevent damage of the cartridge 1 that is caused by contact between the magnets 71 and the cartridge 1, the oscillation mechanism 75 causes the magnets 71 to oscillate within a range in which the magnets 71 do not come into contact with the cartridge 1.

The oscillating rotary shaft 75B is a rotary shaft of the arm 72. The oscillation rotary shaft 75B is in parallel with the horizontal direction so as to be able to cause the magnets 71 to oscillate in the front-back direction. When being viewed from the right or left, the oscillating rotary shaft 75B is disposed in a position that departs from the cartridge 1 toward the front or back of the cartridge. Therefore, when the carriage 73A moves to the lower side, the contact between the cartridge 1 and the arm 72 can be avoided. If the magnets 71 can be caused to oscillate in the front-back direction, the oscillating rotary shaft 75B may be a shaft that is in parallel with the vertical direction.

### 3. Pushing mechanism 80

The pushing mechanism 80 is a mechanism for pushing the plunger 10 of the cartridge 1. When the plunger 10 is pushed by the pushing mechanism 80, the reaction solution plug 47 and oil plug of the cartridge 1 are pushed out to the PCR container 30, whereby the reaction solution 47 in the form of a droplet is formed in the oil of the PCR container 30.

The pushing mechanism 80 has a motor for a plunger 81 and a rod 82. The motor for a plunger 81 is a source of power for moving the rod 82. The rod 82 is a member for pushing the mounting board 11A of the plunger 10 of the cartridge 1. The reason why the mounting board 11A is pushed instead of the tank 3 of the cartridge 1 is that the tank 3 is constituted with a flexible resin that can dilate. When the tank 3 is not deformed, the pushing mechanism 80 may push the tank 3 to push the plunger 10.

The plunger 10 is pushed by the rod 82, not in the vertical direction but in a direction that inclines from the vertical direction by 45°. Accordingly, when the plunger 10 is pushed by the pushing mechanism 80, in the PCR device 50, the rotary body 61 is caused to rotate by 45° to match the longitudinal direction of the cartridge 1 with the movement direction of the rod 82, and then the rod 82 is moved. Since the rod 82 pushes the plunger 10 in the direction that inclines from the vertical direction by 45°, it is easy to dispose the pushing mechanism 80 such that this mechanism becomes free from interference of the elevating mechanism 73. Moreover, since the rod 82 pushes the plunger 10 in the direction that inclines from the vertical direction by 45°, the size of the PCR device 50 can be reduced in the vertical direction.

### 4. Fluorescence measuring instrument 55

The fluorescence measuring instrument 55 is an instrument for measuring luminance of the reaction solution 47 of the PCR container 30. The fluorescence measuring instrument 55 is disposed below the rotary body 61 so as to face the bottom 35A of the PCR container 30 of the cartridge 1. The fluorescence measuring instrument 55 measures luminance of the reaction solution 47 present in the bottom 35A of the PCR container 30 from the opening of the lower side of the insertion hole 64A of the low temperature-side heater 65C. It is desirable that the fluorescence measuring instrument 55 can detect luminance of plural wavelength regions such that the PCR device 50 can be used for multiplex PCR.

### 5. Controller 90

The controller 90 is a portion for controlling the PCR device 50. The controller 90 has, for example, a processor such as CPU and a memory such as ROM or RAM. In the memory, various programs and data are stored. Moreover, the memory provides a region for running the programs. When the processor executes the program stored in the memory, various types of process is performed.

For example, the controller 90 controls the motor for rotation 66 to rotate the rotary body 61 to a predetermined rotation position. The rotation mechanism 60 is provided with a rotation position sensor not shown in the drawing, and the controller 90 drives and stops the motor for rotation 66 in response to the results detected by the rotation position sensor.

Furthermore, the controller 90 controls the heaters (heater for elution 65A, high temperature-side heater 65B, and low temperature-side heater 65C) such that each heater generates heat. The heat block constituting the heaters is provided with a temperature sensor not shown in the drawing. The controller 90 controls ON and OFF of the cartridge heater in response to the results detected by the temperature sensor.

In addition, the controller 90 controls the motor for elevation 73B to move the magnets 71 in the vertical direction. The PCR device 50 is provided with a position sensor not shown in the drawing that detects the position of the carriage 73A. The controller 90 drives and stops the motor for elevation 73B in response to the results detected by the position sensor.

Moreover, the controller 90 controls the motor for oscillation 75A to cause the magnets 71 to oscillate in the front-back direction. The PCR device 50 is provided with a position sensor that detects the position of the arm 72 holding the magnets 71. The controller 90 drives and stops the motor for oscillation 75A in response to the results detected by the position sensor.

The controller 90 controls the fluorescence measuring instrument 55 to measure luminance of the reaction solution 47 of the PCR container 30. When the fluorescence measuring instrument 55 faces the bottom 35A of the PCR container 30 of the cartridge 1, the controller 90 controls the fluorescence measuring instrument 55 to measure the luminance. The measurement results are stored in the memory.

### Description of operation

### 1. Operation for mounting cartridge 1

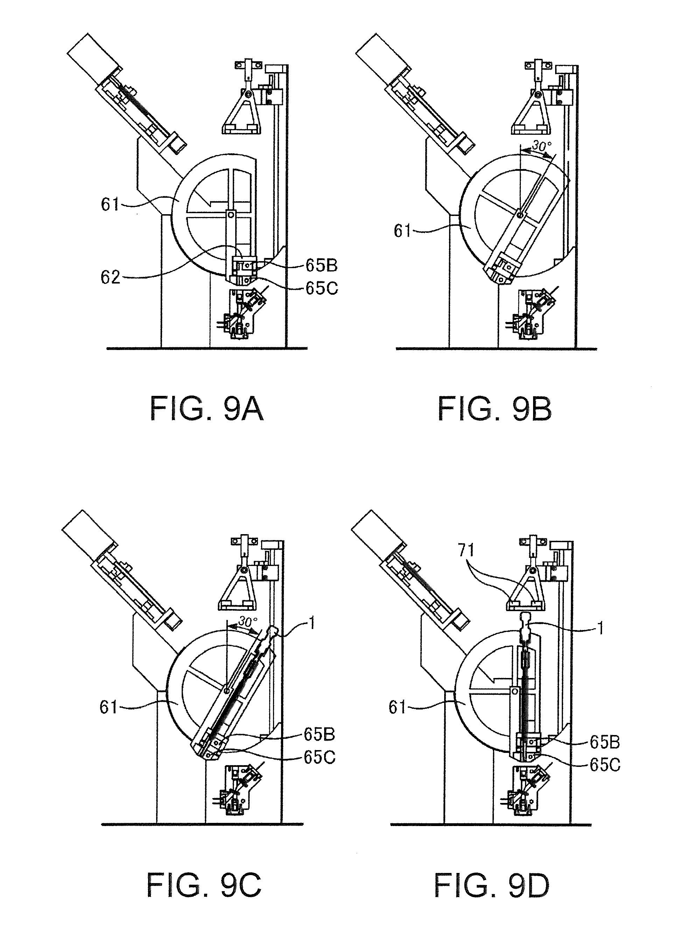

Figs. 9A to 9D are views for illustrating the state of the PCR device 50 at the time of mounting the cartridge 1. Fig. 9A is a view for illustrating an initial state where the cartridge 1 has not yet been mounted. Fig. 9B is a view for illustrating a stand-by state. Fig. 9C is a view for illustrating the state where the cartridge 1 has just been mounted. Fig. 9D is a view for illustrating an initial state in the state where the cartridge 1 has been mounted.

As shown in Fig. 9A, in the initial state where the cartridge 1 has not yet been mounted, the mounting direction of the mounting portion 62 is in the vertical direction. In the following description, the rotation position of the rotary body 61 in this state is taken as a standard (0°), and if the rotary body 61 rotates in a counter clockwise direction when viewed from the right, the direction is regarded as being a positive direction to describe the rotation position of the rotary body 61.

As shown in Fig. 9B, the controller 90 drives the motor for rotation 66 to rotate the rotary body 61 by -30°. In this state, an operator inserts the cartridge 1 into the mounting portion 62 from the cartridge insertion port 53A. At this time, since the cartridge 1 is inserted into the mounting portion 62 while the guide plate 26 is being guided by the guide rail 63A, the PCR container 30 of the cartridge 1 is guided to the insertion hole 64A of the mounting portion 62. The operator inserts the cartridge 1 until the fixing claw 25 of the cartridge 1 is hooked on the notch of the fixing portion 63. In this manner, the cartridge 1 is fixed to a normal position of the mounting portion 62. When the PCR container 30 is not inserted into the insertion hole 64A, and the cartridge 1 is fixed to an abnormal position of the mounting portion 62, the fixing claw 25 of the cartridge 1 is not hooked on the notch of the fixing portion 63. Accordingly, the operator can see that the cartridge 1 is in an abnormal position.

As shown in Fig. 9C, when the cartridge 1 is fixed to a normal position of the mounting portion 62, the reaction solution plug 47 of the tube 20 faces the heater for elution 65A, the upstream side (high-temperature region 36A) of the flow path formation portion 35 of the PCR container 30 faces the high temperature-side heater 65B, and the downstream side (low-temperature region 36B) of the flowpath formation portion 35 of the PCR container 30 faces the low temperature-side heater 65C. Since the mounting portion 62 and the heaters are disposed in the rotary body 61, even when the rotary body 61 rotates, the positional relationship between the cartridge 1 and the heaters is maintained in this state.

After the cartridge 1 is mounted on the mounting portion 62, the controller 90 controls the rotary body 61 to rotate by 30° as shown in Fig. 9D such that the rotary body 61 returns to the standard position. The controller 90 may detect the state where the cartridge 1 has been mounted on the mounting portion 62 by using a sensor not shown in the drawing. Alternately, the controller 90 may detect the state by an input operation performed by the operator.

### 2. Nucleic acid elution process

### Vertical movement of magnets 71

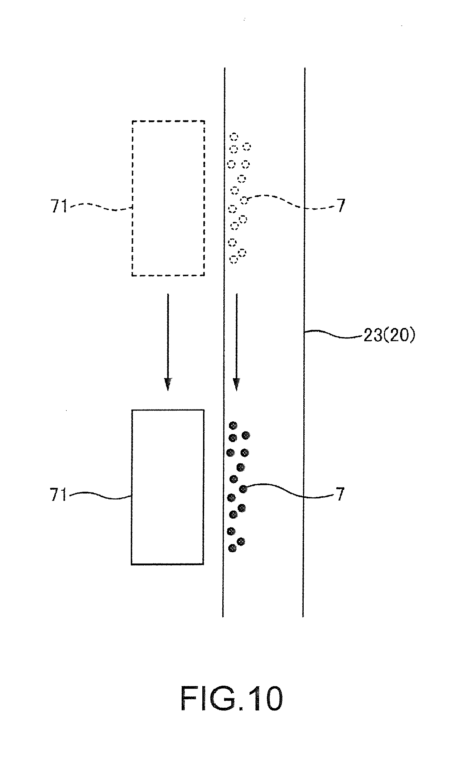

Fig. 10 is a schematic view showing the behavior of the magnetic beads 7 at the time when the magnets 71 are vertically moved. The magnetic beads 7 in the cartridge 1 are attracted to the magnets 71. Accordingly, when the magnets 71 move at the outside of the cartridge 1, the magnetic beads 7 in the cartridge 1 also move along with the magnets 71.

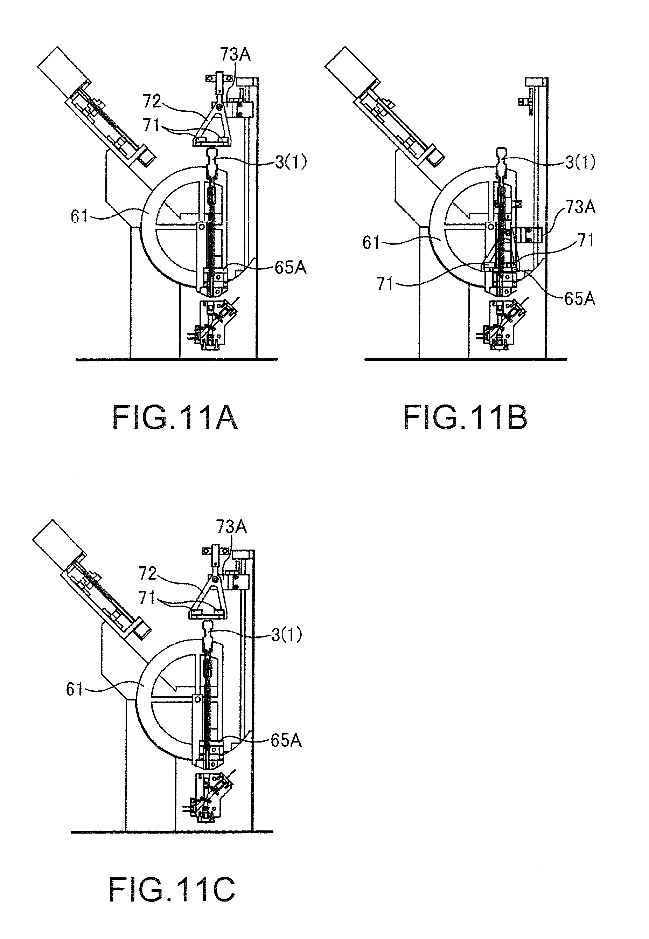

Figs. 11A to 11C are views for illustrating nucleic acid elution process. Fig. 11A is a view for illustrating the state of the PCR device 50 in which the nucleic acid elution process has not yet been performed. Fig. 11B is a view for illustrating the state of the PCR device 50 in which the magnets 71 have been moved to the reaction solution plug 47. Fig. 11C is a view for illustrating the state of the PCR device 50 in which the magnets 71 have been drawn up.

As shown in Fig. 11A, in the initial state, the tank 3 becomes the upper side of the cartridge 1, and the longitudinal direction of the cartridge 1 is in parallel with the vertical direction of the PCR device 50. In this state, as shown in Fig. 2A, the cartridge 1 has the lysing solution 41 (tank 3) containing the magnetic beads 7, the oil 42 (plunger 10), the washing solution 43 (upstream side of the tube 20), the first oil plug 44 (capillary 23), the washing solution plug 45 (capillary 23), the second oil plug 46 (capillary 23), the reaction solution plug 47 (capillary 23), the third oil plug 48 (capillary 23), and oil (PCR container 30) in this order from the upper side of the cartridge.

As shown in Fig. 11A, in the initial state, the carriage 73A is in the uppermost position (retraction position), and the magnets 71 are placed above the cartridge 1. In this state, the controller 90 drives the motor for elevation 73B to slowly move downward the carriage 73A such that the magnets 71 slowly move downward. Since the longitudinal direction of the cartridge 1 is in parallel with the vertical direction of the PCR device 50, the magnets 71 move along the cartridge 1.

When the magnets 71 move downward, the magnets 71 face the tank 3, and the magnetic beads 7 in the tank 3 are attracted to the magnets 71. The controller 90 moves downward the carriage 73A at such a speed that enables the magnetic beads 7 to move together with the magnets 71.

When the magnets 71 move from the position (the section of the tank 3) facing the tank 3 to the position (the section of the plunger 10) facing the plunger 10, the magnetic beads 7 pass through the opening of the upstream side of the cylindrical portion 11 of the plunger 10 and then pass through the interface between the lysing solution 41 in the tank 3 and the oil 42 of the upstream side of the cartridge main body 9. As a result, the magnetic beads 7 having bound to the nucleic acid are introduced into the cartridge main body 9. When the magnetic beads 7 pass through the interface between the lysing solution 41 and the oil 42, the lysing solution 41 is wiped by the oil 42. Consequently, the components of the lysing solution 41 are not easily brought into the oil 42. As a result, it is possible to prevent the components of the lysing solution 41 from being mixed into the washing solution or the reaction solution 47.

When the magnets 71 move downward in the state of facing the plunger 10, the magnetic beads 7 pass through the inside of the cylindrical portion 11, escape from the inside and outside of the ribs 13, come out of the opening of the downstream side of the cylindrical portion 11, and are introduced into the upper syringe 21 of the tube 20. Meanwhile, the magnetic beads 7 pass through the interface between the oil 42 and the washing solution 43, in the plunger 10. When the magnetic beads 7 are introduced into the washing solution 43, the nucleic acid having bound to the magnetic beads 7 is washed with the washing solution 43.

At this stage, the rod-like portion 12 of the plunger 10 has not yet been inserted into the lower syringe 22 of the tube 20. Accordingly, when the magnets 71 move from the position (the section of the upper syringe 21) facing the upper syringe 21 to the position (the section of the capillary 23) facing the capillary 23, the magnetic beads 7 move to the lower syringe 22 from the upper syringe 21 and then move to the capillary 23 from the lower syringe 22. The first oil plug 44 is at the upstream side of the capillary 23, and when the magnetic beads 7 move to the capillary 23 from the lower syringe 22, the magnetic beads 7 pass through the interface between the washing solution 43 and the oil. At this time, since the washing solution 43 is wiped by the oil, the components of the washing solution 43 are not easily brought into the oil. In this manner, it is possible to prevent the components of the washing solution 43 from being mixed into the washing solution plug 45 or the reaction solution plug 47.

When the magnets 71 move from the position (the section of the first oil plug 44) facing the first oil plug 44 to the position (the section of the washing solution plug 45) facing the washing solution plug 45, the magnetic beads 7 pass through the interface between the oil and the washing solution. When the magnetic beads 7 are introduced into the washing solution plug 45, the nucleic acid having bound to the magnetic beads 7 is washed with the washing solution.

When the magnets 71 move from the position (the section of the washing solution plug 45) facing the washing solution plug 45 to the position (the section of the second oil plug 46) facing the second oil plug 46, the washing solution and the magnetic beads 7 pass through the interface between the washing solution and the oil. At this time, since the washing solution is wiped by the oil, the components of the washing solution are not easily brought into the oil. In this manner, it is possible to prevent the components of the washing solution from being mixed into the reaction solution plug 47.

When the magnets 71 move from the position (the section of the second oil plug 46) facing the second oil plug 46 to the position (the section of the reaction solution plug 47) facing the reaction solution plug 47, the magnetic beads 7 pass through the interface between the oil and the reaction solution 47.

Before the magnetic beads 7 are introduced into the reaction solution plug 47, the controller 90 controls the heater for elution 65A to heat the reaction solution plug 47 to about 50°C. Moreover, if the reaction solution 47 is heated before the magnetic beads 7 are introduced into the reaction solution 47, the time from when the magnetic beads 7 are introduced into the reaction solution 47 to when the elution of the nucleic acid is completed can be shortened.

As shown in Fig. 11B, after the magnets 71 move to the position (the section of the reaction solution plug 47) facing the reaction solution plug 47, the controller 90 stops the motor for elevation 73B to stop the movement of the magnets 71 in the vertical direction and heats the reaction solution plug 47 for 30 seconds at 50°C. As a result, the nucleic acid having bound to the magnetic beads 7 is liberated into the solution of the reaction solution plug 47, and a reverse transcription reaction is caused. If the reaction solution 47 is heated, the elution of nucleic acid from the magnetic beads 7 and the reverse transcription reaction are accelerated.

After the elution of nucleic acid is caused in the reaction solution plug 47, the controller 90 drives the motor for elevation 73B in a direction opposite to the direction in which the motor has been driven, such that the carriage 73A slowly moves upward to slowly move the magnets 71 upward. The controller 90 moves the carriage 73A upward at such a speed that enables the magnetic beads 7 to move together with the magnets 71.

When the magnets 71 move upward in the state shown in Fig. 11B, the magnetic beads 7 move to the second oil plug 46 from the reaction solution plug 47, whereby the magnetic beads 7 are removed from the reaction solution plug 47.

When the magnets 71 slowly move to the position facing the upper syringe 21, the magnetic beads 7 also move to the upper syringe 21 and are positioned above the lower syringe 22. If moved to such a position, the magnetic beads 7 are never introduced into the PCR container 30 when the plunger 10 is pushed. Therefore, while the state of the PCR device 50 is being changed to the state shown in Fig. 11C from the state described above, the controller 90 may move the carriage 73A upward at such a speed that inhibits the magnetic beads 7 from following the movement of the magnets 71. In addition, if the magnetic beads 7 are not introduced into the PCR container 30 when the plunger 10 is pushed, the movement speed of the carriage 73A may be increased at the earlier stage.

The memory of the controller 90 stores information regarding the movement speed of the magnets 71. The controller 90 executes the above operation (operation for causing the magnets 71 to move up and down) according to the information.

### Oscillation of magnets 71

While the magnets 71 are being moved vertically, the controller 90 may drive the motor for oscillation 75A such that the pair of magnets 71 sandwiching the cartridge 1 therebetween oscillates in the front-back direction.

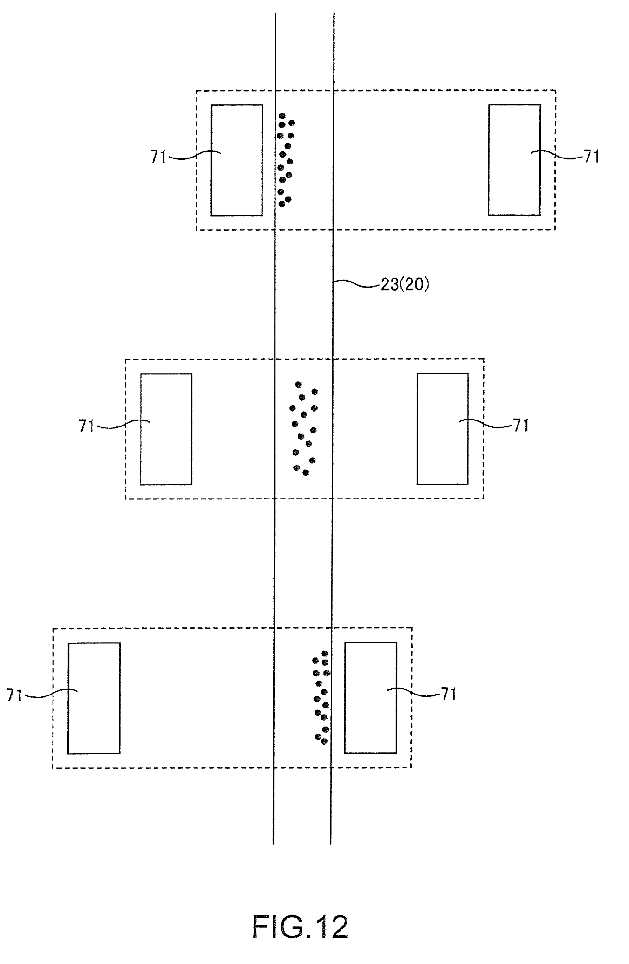

Fig. 12 is a schematic view showing the behavior of the magnetic beads 7 at the time when the magnets 71 are caused to oscillate.

While the magnets 71 are moving in the vertical direction, the tube 20 is interposed between the pair of magnets 71 in the front-back direction. Since the pair of magnets 71 is held by the arm 72, the distance between the pair of magnets 71 in the front-back direction is almost constant. Therefore, one of the pair of magnets 71 approaches the tube 20, and the other magnet is separated from the tube 20.

The magnetic beads 7 are attracted to the magnet 71 that is close to the magnetic beads. Accordingly, when one of the pair of magnets 71 approaches the tube 20, the magnetic beads 7 are attracted to the approaching magnet 71. Thereafter, when the above magnet 71 is separated from the tube 20, and then the other magnet 71 approaches the tube 20, the magnetic beads 7 are attracted to the approaching magnet 71. In this manner, the magnetic beads 7 move in the front-back direction. When the pair of magnets 71 is caused to oscillate in the front-back direction, the magnetic beads 7 reciprocate in the front-back direction.

When the magnetic beads 7 reciprocate in the front-back direction, the magnetic beads 7 easily come into contact with liquid. Particularly, since the liquid in the capillary 23 practically does not exhibit fluidity, when it is desired to make the liquid in the capillary 23 become close to the magnetic beads 7 as much as possible, it is effective to cause the magnetic beads 7 to reciprocate in the front-back direction.

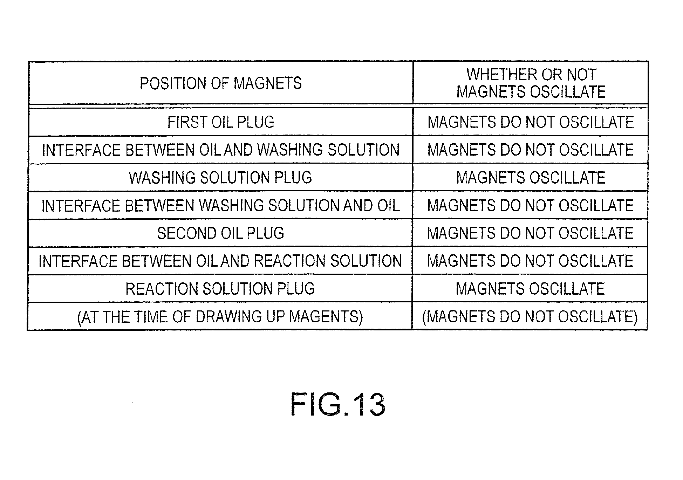

Fig. 13 is a table showing whether or not the magnets 71 oscillate.

When the magnetic beads 7 move the oil plug (the first oil plug 44 or the second oil plug 46) downward, the controller 90 stops the oscillation motor such that the magnets 71 do not oscillate. At this time, the controller 90 moves the magnets 71 downward, in a state where one of the pair of magnets 71 has been brought close to the tube 20. This is because the magnetic beads 7 more easily follow the movement of the magnets 71, compared to the case where the respective magnets 71 are separated from the tube 20 by the same distance.

When the magnetic beads 7 move the washing solution plug 45 downward, the controller 90 drives the oscillation motor to cause the magnets 71 to oscillate in the front-back direction. As a result, the magnetic beads 7 move downward while oscillating in the front-back direction inside the washing solution plug 45, hence the washing effect of the magnetic beads 7 can be enhanced. Moreover, since the washing effect is enhanced, the amount of the washing solution plug 45 can be reduced, and the cartridge 1 can be miniaturized.

When the magnetic beads 7 pass through the interface between the washing solution and oil (second oil plug 46), the controller 90 stops the oscillation motor such that the magnets 71 do not oscillate. In this manner, since the magnetic beads 7 do not oscillate when passing through the interface, the components of the washing solution are not easily brought into the oil. Moreover, the controller 90 moves the magnets 71 downward, in a state where one of the pair of magnets 71 is brought close to the tube 20. As a result, the magnetic beads 7 close to the magnet 71 are attracted to the magnet and are aggregated, and the washing solution having adhered to the magnetic beads 7 is squeezed out, hence the components of the washing solution are not easily brought into the oil.

When the magnetic beads 7 are in the reaction solution plug 47, the controller 90 drives the oscillation motor such that the magnets 71 oscillate in the front-back direction. In this manner, since the magnetic beads 7 oscillate in the front-back direction inside the reaction solution plug 47, the elution effect of the nucleic acid having bound to the magnetic beads 7 can be enhanced. Moreover, since the elution effect is enhanced, the time from when the magnetic beads 7 are introduced into the reaction solution 47 to when the elution of the nucleic acid is completed can be shortened.

When the nucleic acid is eluted in the reaction solution plug 47, and then the magnets 71 are moved upward to draw up the magnetic beads 7, the controller 90 stops the oscillation motor such that the magnets 71 do not oscillate. At this time, the controller 90 moves the magnets 71 downward, in a state where one of the pair of magnets 71 is brought close to the tube 20. As a result, the magnetic beads 7 easily follow the movement of the magnets 71, and the movement speed of the magnets 71 can be increased.

The memory of the controller 90 stores the information regarding the position of each plug of the capillary 23 and the information regarding oscillation as shown in Fig. 13. According to the information, the controller 90 executes the aforementioned operation (operation for causing the magnets 71 to oscillate).

### 3. Solution droplet formation process

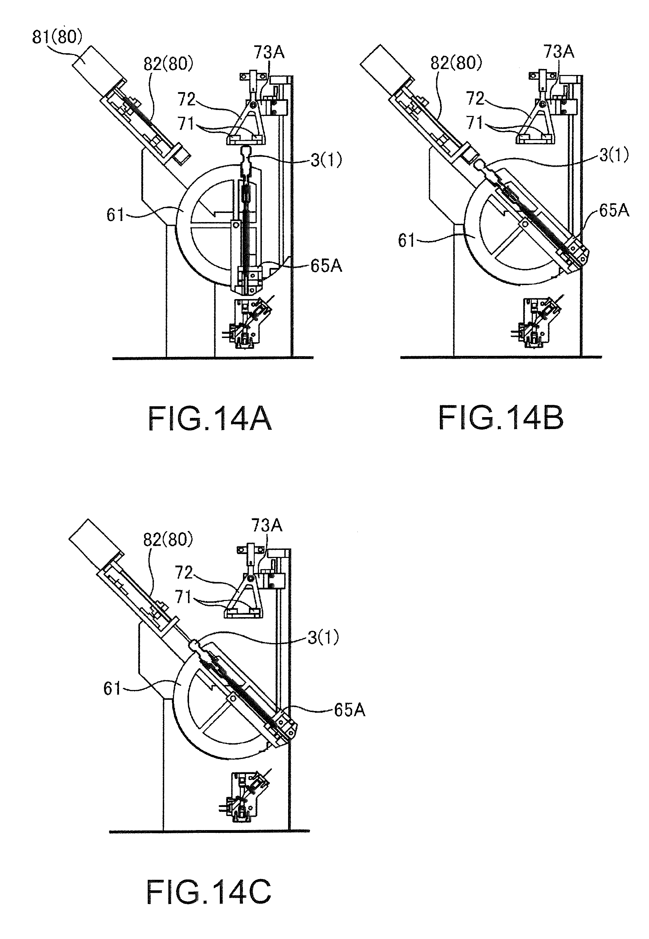

Figs. 14A to 14C are views for illustrating solution droplet formation process. Fig. 14A is a view for illustrating the state of the PCR device 50 at the time when the magnets 71 have been drawn up. Fig. 14B is a view for illustrating the state where the rotary body 61 has been rotated by 45°. Fig. 14C is a view for illustrating the state where the rod 82 of the pushing mechanism 80 has pushed the plunger 10.

As shown in Fig. 14A, when the carriage 73A is in the retraction position, the elevating mechanism 73 does not come into contact with the cartridge 1 even when the cartridge 1 rotates. After the PCR device 50 is put in this state, the controller 90 rotates the rotary body 61 by 45°.

As shown in FIG. 14B, when the rotary body 61 rotates by 45°, the longitudinal direction of the cartridge 1 is in parallel with the movement direction of the rod 82 of the pushing mechanism 80. The controller 90 drives the motor for a plunger 81 to move the rod 82. When the rod 82 comes into contact with the mounting board 11A of the plunger 10 of the cartridge 1 and then moves further, the plunger 10 is pushed into the tube 20. The controller 90 moves the rod 82 until the state shown in Fig 14C is created and pushes the plunger 10 until the mounting board 11A of the plunger 10 comes into contact with the upper rim of the tube 20.

When the plunger 10 is pushed into the tube 20, the seal 12A of the rod-like portion 12 of the plunger 10 fits to the lower syringe 22 of the tube 20 (see Fig. 2B). Thereafter, when the plunger 10 is further pushed, the seal 12A slides inside the lower syringe 22. As a result, the liquid (the third oil plug 48, the reaction solution plug 47, and the like) of the downstream side of the tube 20 is pushed out to the flow path formation portion 35 of the PCR container 30, in such an amount corresponding to the volume in which the seal 12A slides inside the lower syringe 22.

First, the third oil plug 48 of the tube 20 flows into the flow path formation portion 35. Since the flow path formation portion 35 is filled with oil, the inflow oil flows into the oil accommodating space 32A from the flow path formation portion 35, whereby the oil interface of the oil accommodating space 32A ascends. At this time, due to pressure loss of the lower seal portion 34B, the pressure of the liquid of the flow path formation portion 35 becomes higher than the outside pressure (pressure of the oil accommodating space 32A) . After the third oil plug 48 is pushed out of the tube 20, the reaction solution plug 47 flows into the flow path formation portion 35 from the tube 20. Since the inner diameter of the flow path formation portion 35 is larger than the inner diameter of the capillary 23, the reaction solution 47, which has been in the form of a plug in the tube 20, is formed into a droplet in the oil of the flow path formation portion 35.

The volume in which the seal 12A slides inside the lower syringe 22 (the amount of the liquid in the tube 20 that is pushed out from the downstream side) is larger than the total volume of the reaction solution plug 47 and the third oil plug 48 in the tube 20. Therefore, after the reaction solution plug 47 is pushed out of the tube 20, a portion of the second oil plug 46 is also pushed out to the flow path formation portion 35. As a result, the reaction solution 47 does not remain in the tube 20, and the entire reaction solution plug 47 is formed into a droplet. Moreover, since a portion of the second oil plug 46 is pushed out of the downstream side of the tube 20, the reaction solution 47 in the form of a droplet easily leaves the tube 20 (the reaction solution 47 in the form of a droplet is not easily adsorbed onto the opening of the capillary 23).

The capillary 23 is designed such that the inner diameter of the terminal thereof (diameter of the opening of the capillary 23) is relatively small. Therefore, the reaction solution 47, which has been formed into a droplet in the PCR container 30, is not easily adsorbed onto the opening of the capillary 23. Moreover, the specific gravity of the reaction solution 47 is greater than that of the oil of the PCR container 30. Consequently, the reaction solution 47 in the form of a droplet leaves the terminal of the capillary 23, passes through the flow path formation portion 35 as a flow path, and is precipitated to the bottom 35A. Here, at this stage, since the flow path of the flow path formation portion 35 inclines by 45°, the reaction solution 47 in the form of a droplet easily adheres to the inner wall of the flow path formation portion 35. Therefore, the flow path of the flow path formation portion 35 needs to be returned to the vertical direction.

After the reaction solution 47 in the form of a droplet is formed (after the plunger 10 is pushed), the controller 90 drives the motor for a plunger 81 in a direction opposite to the direction in which the motor has been driven, such that the rod 82 returns to the original position. In this state, even if the cartridge 1 rotates, the rod 82 of the pushing mechanism 80 does not come into contact with the cartridge 1. After the PCR device 50 is put in this state, the controller 90 returns the rotary body 61 to the standard position. When the rotary body 61 returns to the standard position, the flow path of the flow path formation portion 35 is in the vertical direction. Accordingly, the reaction solution 47 in the form of a droplet does not easily adhere to the inner wall of the flow path formation portion 35.

### 4. Thermal cycling process

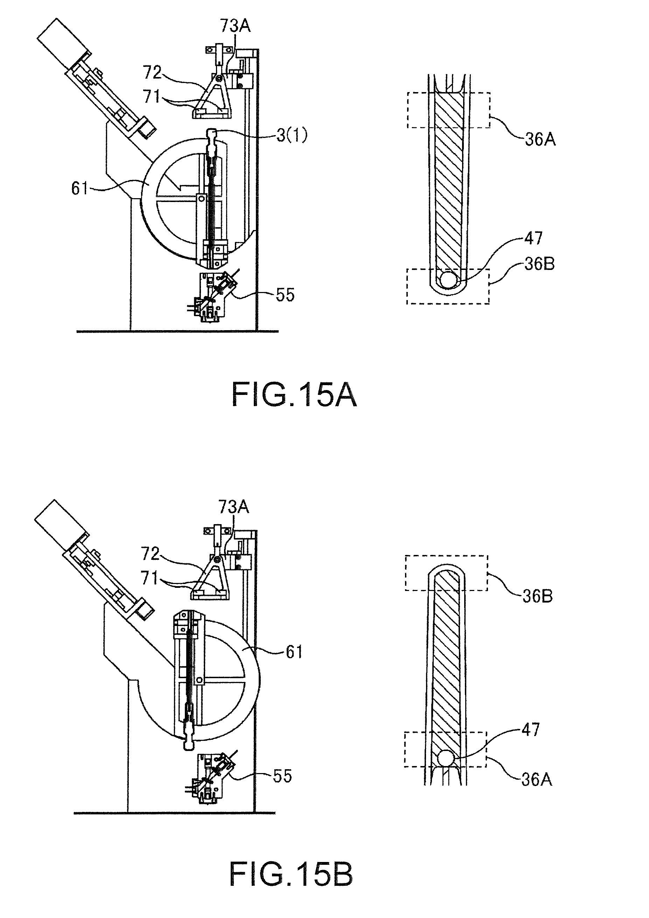

Figs. 15A and 15B are views for illustrating thermal cycling process. Fig. 15A is a view for illustrating the state where the reaction solution 47 is subjected to temperature process at a low-temperature side. Fig. 15B is a view for illustrating the state where the reaction solution 47 is subjected to temperature process at a high-temperature side. The left side of each drawing describes the state of the PCR device 50, and the right side of each drawing describes the internal state of the flow path formation portion 35 of the PCR container 30.

When the cartridge 1 is fixed to a normal position in the mounting portion 62, the upstream side (high-temperature region 36A) of the flow path formation portion 35 of the PCR container 30 faces the high temperature-side heater 65B, and the downstream side (low-temperature region 36B) of the flow path formation portion 35 of the PCR container 30 faces the low temperature-side heater 65C. During the thermal cycling process, the controller 90 controls the high temperature-side heater 65B disposed in the rotary body 61, such that the liquid of the high-temperature region 36A of the upstream side of the flow path formation portion 35 of the PCR container 30 is heated to about 90°C to 100°C. Moreover, the controller 90 controls the low temperature-side heater 65C disposed in the rotary body 61, such that the liquid of the low-temperature region 36B of the downstream side of the flow path formation portion 35 is heated to about 50°C to 75°C. As a result, during the thermal cycling process, a temperature gradient is formed in the liquid in the flow path formation portion 35 of the PCR container 30. Since the mounting portion 62 and the heaters are disposed in the rotary body 61, the positional relationship between the cartridge 1 and the heaters is maintained in this state even when the rotary body 61 rotates.

During the thermal cycling process, the liquid in the PCR container 30 is heated. If air bubbles are formed in the liquid of the PCR container 30 due to heating of the liquid, the temperature of the liquid in the flow path formation portion 35 may become uneven, or movement (precipitation) of the reaction solution 47 in the form of a droplet that is caused in the flow path formation portion 35 may be hindered. However, in the present embodiment, due to pressure loss of the lower seal portion 34B, the pressure of the liquid of the flow path formation portion 35 is higher than the outside pressure. Accordingly, air bubbles are not easily formed in the liquid of the PCR container 30.

As shown in Fig. 15A, when the rotary body 61 is in the standard position, the low temperature-side heater 65C is positioned below the high temperature-side heater 65B, and the bottom 35A of the PCR container 30 of the cartridge 1 becomes the lower side. Since the specific gravity of the reaction solution 47 in the form of a droplet is greater than that of oil, the reaction solution 47 in the form of a droplet is precipitated inside the flow path formation portion 35. After being precipitated inside the flow path formation portion 35, the reaction solution 47 in the form of a droplet reaches the bottom 35A of the PCR container 30. The precipitation of the reaction solution 47 is stopped at the bottom 35A, and the reaction solution stays in the low-temperature region 36B. In this manner, the reaction solution 47 in the form of a droplet moves to the low-temperature region 36B. The controller 90 maintains the state shown in Fig. 15A for a predetermined time and heats the reaction solution 47 in the form of a droplet to about 50°C to 75°C in the low-temperature region 36B (the reaction solution 47 is subjected to temperature process at the low-temperature side). Meanwhile, an extension reaction of the polymerase reaction occurs.

When the controller 90 drives the motor for rotation 66 in the state shown in Fig. 15A to rotate the rotary body 61 by 180°, the state shown in Fig 15B is created. When the rotary body 61 rotates by 180° from the standard position, the cartridge 1 becomes upside down, and the high temperature-side heater 65B as well as the low temperature-side heater 65C also become upside down. That is, the high temperature-side heater 65B is positioned below the low temperature-side heater 65C, and the bottom 35A of the PCR container 30 of the cartridge 1 becomes the upper side. After being precipitated inside the flow path formation portion 35, the reaction solution 47 in the form of a droplet reaches the terminal of the tube 20 (terminal of the capillary 23). The precipitation of the reaction solution 47 is stopped at the terminal, and the reaction solution 47 stays in the high-temperature region 36A. In this manner, the reaction solution 47 in the form of a droplet moves to the high-temperature region 36A. The controller 90 maintains the state of Fig. 15B for a predetermined time, and heats the reaction solution 47 in the form of a droplet to about 90°C to 100°C in the high-temperature region 36A (the reaction solution 47 is subjected to temperature process at the high-temperature side). Meanwhile, a denaturation reaction of the polymerase reaction occurs.

When the controller 90 drives the motor for rotation 66 in the state of Fig. 15B to rotate the rotary body 61 by -180°, the PCR device 50 returns to the state of Fig. 15A. When being precipitated inside the flow path formation portion 35 in this state, the reaction solution 47 in the form of a droplet moves to the low-temperature region 36B and is heated again to about 50°C to 75°C in the low-temperature region 36B (the reaction solution 47 is subjected to temperature process at the low-temperature side). Since the capillary 23 is designed such that the inner diameter of terminal thereof (diameter of the opening of the capillary 23) is relatively small, the reaction solution 47 is not easily adsorbed onto the opening of the capillary 23. Therefore, when the rotary body 61 rotates by -180° in the state of Fig. 15B, the reaction solution 47 in the form of a droplet leaves the tube 20 and is precipitated to the bottom 35A of the PCR container 30, without being adsorbed onto the opening of the capillary 23.

The controller 90 drives the motor for rotation 66 such that the rotation position of the rotary body 61 is put in the state of Fig. 15A and the state of Fig. 15B. The controller 90 repeats this operation for a predetermined cycle number. As a result, the PCR device 50 can perform the thermal cycling process of PCR on the reaction solution 47.

The memory of the controller 90 stores the temperature of the high temperature-side heater 65B, the temperature of the low temperature-side heater 65C, the time during which the PCR device 50 is held in the state of Fig. 15A, the time during which the PCR device 50 is held in the state of Fig. 15B, and thermal cycling information of the cycle number (the number of times of repetition of the state of Fig. 15A and the state of Fig. 15B). According to the thermal cycling information, the controller 90 executes the above process.

### 5. Fluorescence measurement

As shown in Fig. 15A, when the rotary body 61 is in the standard position, the fluorescence measuring instrument 55 faces the bottom 35A of the PCR container 30 of the cartridge 1. Accordingly, at the time of performing fluorescence measurement on the reaction solution 47, the controller 90 controls the fluorescence measuring instrument 55 to measure fluorescence intensity of the reaction solution 47, which is in the bottom 35A of the PCR container 30, from the opening of the lower side of the insertion hole 64A of the low temperature-side heater 65C, in the state where the rotary body 61 is in the standard position.

Immediately after the rotary body 61 rotates by 180° and reaches the standard position, the reaction solution 47 in the form of a droplet is being precipitated in the flow path formation portion 35 of the PCR container 30 in some cases, hence the reaction solution 47 in the form of a droplet has not yet reached the bottom 35A of the PCR container 30. Therefore, it is desirable for the controller 90 to perform the fluorescence intensity after a predetermined time elapses from when the rotary body 61 is placed in the rotation position as shown in Fig. 15A (immediately before the rotary body 61 is rotated in the state of Fig. 15A). Alternatively, the controller 90 may control the fluorescence measuring instrument 55 to measure the fluorescence intensity during a predetermined period of time from when the rotary body 61 is placed in the standard position, and may store the time history of the fluorescence intensity.

### Overview

As described above, the PCR device 50 as a nucleic acid amplification reaction device has a rotary body 61 that includes a mounting portion (consisting of the fixing portion 63 and the insertion hole 64A) on which the cartridge 1 is mounted, and heaters for PCR (the high temperature-side heater 65B and the low temperature-side heater 65C) that forms a temperature gradient in the inside of the PCR container 30 as a nucleic acid amplification reaction container. The cartridge 1 mounted on the rotary body 61 has the tube 20 that has the reaction solution plug 47 containing an eluate, and the PCR container 30 that contains oil. In the PCR device 50, when the posture of the cartridge 1 is changed due to the rotation of the rotary body 61, the reaction solution 47 in the form of a droplet, which has been introduced from the tube 20, moves inside the PCR container 30. As a result, the posture of the PCR container 30 as well as the posture of the tube 20 in which the nucleic acid elution process is performed are changed at the same time to perform a polymerase reaction. Accordingly, the process time can be shortened.

According to the PCR device 50, the rotary shaft of the rotary body 61 is positioned closer to the tube 20 than to the PCR container 30 of the cartridge 1. Therefore, the rotary body 61 can be miniaturized. Moreover, since the tube 20 is longer than the PCR container 30, if the rotary shaft is positioned at the center of the PCR container 30, the size of the rotary body 61 increases.

The fixing portion 63 to which the tube 20 of the cartridge 1 is fixed and the insertion hole 64A to which the PCR container 30 is fixed function as the mounting portion for mounting the cartridge on the rotary body. Therefore, the long cartridge 1 constituted with the tube 20 and the PCR container 30 is stably fixed.

In the PCR device 50, heaters for PCR (the high temperature-side heater 65B and the low temperature-side heater 65C) are disposed in the rotary body 61. Accordingly, regardless of the rotation position of the rotary body 61, the positional relationship between the PCR container 30 of the cartridge 1 and the heaters for PCR is maintained, and the temperature gradient formed inside the PCR container is stabilized.

The PCR device 50 also includes the heater for elution 65A. Accordingly, the liberation of nucleic acid from magnetic beads is accelerated.

The PCR device 50 has the magnet moving mechanism 70 (elevating mechanism 73) that moves the magnets 71 along the tube 20. Accordingly, the movement of the magnetic beads which are nucleic acid-binding solid-phase carriers can be automated, and the magnetic beads can be moved in the same pattern all the time.

Moreover, the magnet moving mechanism 70 has the oscillation mechanism 75. Accordingly, the aggregation and diffusion of the magnetic beads that are caused inside the tube 20 can be regulated, the washing effect, elution effect, and the like can be enhanced, and the process time can be shortened.

The PCR device 50 also has the pushing mechanism 80 that pushes the plunger 10 of the cartridge 1. Accordingly, the process for introducing the reaction solution plug 47, into which the nucleic acid has been eluted, into the PCR container 30 from the tube 20 can be automated.

### Second embodiment

In a second embodiment, the cartridge 1 having constitution different from that of the first embodiment is mounted on the PCR device 50.

### Cartridge 1

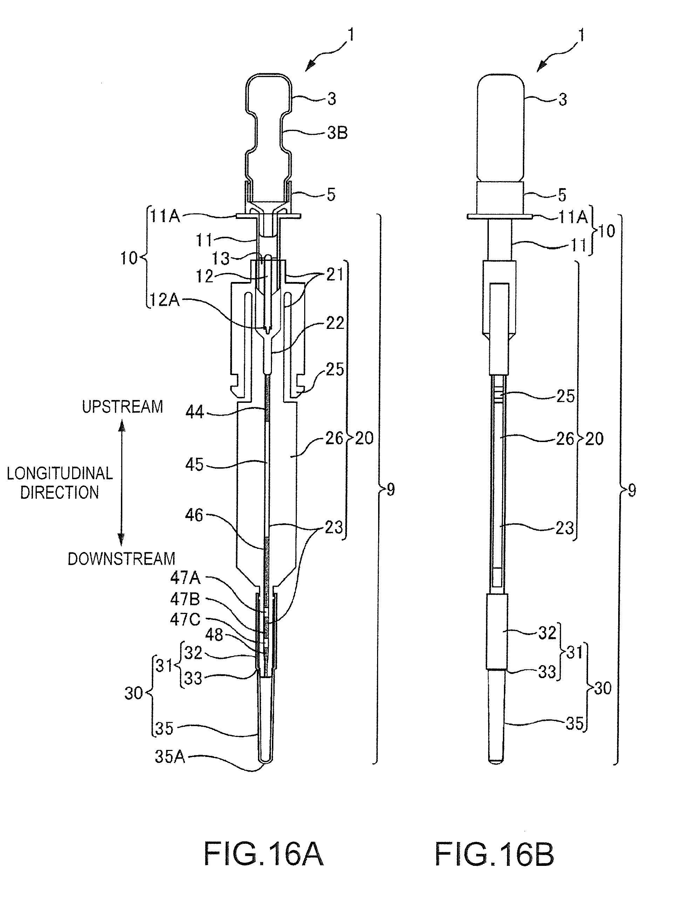

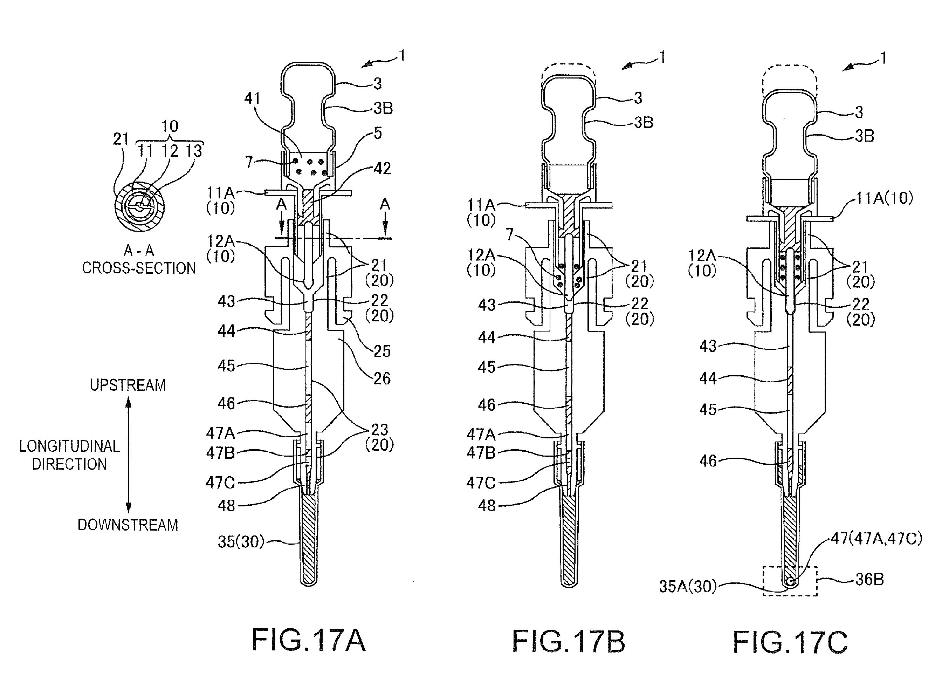

Figs. 16A and 16B are views for illustrating the cartridge 1 of the second embodiment. Fig. 17A is a view for illustrating the initial state of the cartridge 1 of the second embodiment. Fig. 17B is a lateral view showing the state where the plunger 10 has been pushed in the stae of Fig. 17A to bring the seal 12A into contact with the lower syringe 22. Fig. 17C is a view for illustrating the cartridge 1 of the second embodiment in which the plunger 10 has been pushed. The tube 20 of the cartridge 1 of the second embodiment has an eluate plug 47A, an oil plug 47B, and a nucleic acid amplification reaction solution plug 47C, instead of the reaction solution plug 47 of the first embodiment. The oil plug 47B prevents the eluate plug 47A and the nucleic acid amplification reaction solution plug 47C, which are water-soluble plugs at both sides of the oil plug 47B, from being mixed with each other.

The cartridge 1 is a container for performing nucleic acid elution process that causes the nucleic acid to be eluted from the magnetic beads 7 having bound to the nucleic acid and performing thermal cycling process for a polymerase reaction on a PCR solution which is a mixed solution consisting of the eluate 47A and the nucleic acid amplification reaction solution 47C.

The shape of the tank 3 and the cartridge main body 9 of the cartridge 1 is the same as in the first embodiment. Moreover, the lysing solution 41 of the tank 3 of the cartridge 1 is also the same as that of the first embodiment. Furthermore, the oil 42, the first washing solution 43, the first oil plug 44, the washing solution plug 45 (the second washing solution), and the second oil plug 46 of the cartridge main body 9 are the same as those of the first embodiment. Accordingly, these will not be described again.

The eluate plug 47A and the nucleic acid amplification reaction solution plug 47C are composed of, for example, the following reaction solution.

| | Reagent | Added amount (µL) | Concentration | Final concentration | Unit |
|---|---|---|---|---|---|
| Eluate | AMV reverse transcriptase (manufactured by NIPPON GENE CO., LTD.) | 0.2 | 20.0 | 0.1 | units/µL |
| | dNTP | 1.6 | 10.0 | 0.8 | mM |
| | buffer * | 4.0 | | 2.0 | |
| | primer R | 1.0 | 10.0 | 0.5 | µM |
| | BSA | 2.0 | 20.0 | 1.0 | mg/mL |
| | DW | 11.2 | | 5.6 | |
| Nucleic acid amplification reaction solution | Gene Taq NT PCR polymerase (manufactured by NIPPON GENE CO., LTD.) | 0.2 | 5.0 | 0.1 | units/µL |
| | dNTP | 1.0 | 10.0 | 0.5 | mM |
| | buffer * | 4.0 | | 2.0 | |
| | primer F | 1.0 | 10.0 | 0.5 | µM |
| | primer R | 1.0 | 10.0 | 0.5 | µM |
| | probe (Taq man) | 0.4 | 12.5 | 0.2 | µM |
| | BSA | 2.0 | 20.0 | 1.0 | mg/mL |
| | DW | 8.4 | | | |

The eluate refers to liquid into which the nucleic acid having adsorbed onto the nucleic acid-binding solid-phase carriers is eluted from the carrier to perform a reverse transcription reaction. Accordingly, the eluate 47A into which the nucleic acid has been eluted is prepared in advance such that this solution is directly used as a buffer solution for the reverse transcription reaction. Moreover, a nucleic acid amplification reaction solution refers to liquid for performing a polymerase reaction.

The portion of the downstream of the capillary 23 is inserted into the PCR container 30. Therefore, by pushing the nucleic acid amplification reaction solution plug 47C and the eluate plug 47A in the tube 20 out of the tube 20, the nucleic acid amplification reaction solution plug 47C and the eluate plug 47A can be introduced into the PCR container 30.

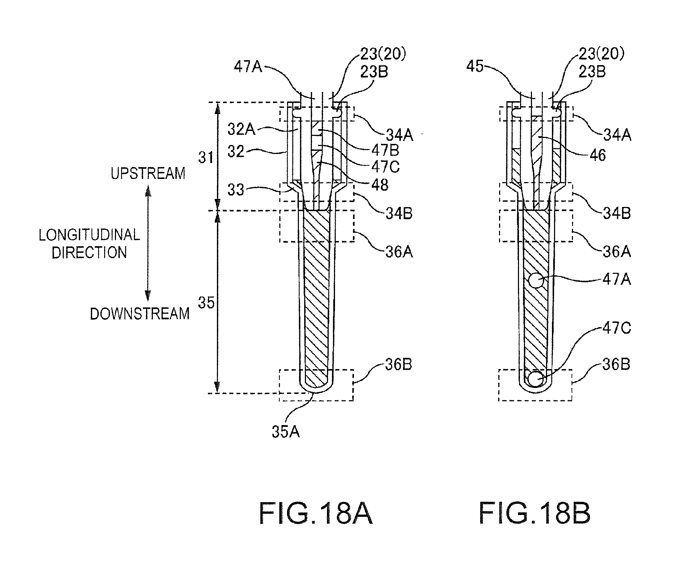

Figs. 18A and 18B are views for illustrating the periphery of the PCR container 30 of the second embodiment. Fig. 18A is a view for illustrating the initial state. Fig. 18B is a view for illustrating the state where the plunger 10 has been pushed. Hereinafter, Figs. 17A to 17C will also be used for describing the PCR container 30.

The PCR container 30 is a container which receives the liquid pushed out of the tube 20 and accommodates a PCR solution as a mixed solution consisting of the nucleic acid amplification reaction solution plug 47C and the eluate plug 47A during the thermal cycling process.

When plunger 10 is pushed, first, the third oil plug 48 of the tube 20 flows into the flow path formation portion 35, the inflow oil flows into the oil accommodating space 32A from the flow path formation portion 35, and the oil interface of the oil accommodating space 32A ascends. At this time, due to pressure loss of the lower seal portion 34B, the pressure of the liquid of the flow path formation portion 35 increases.

After the third oil plug 48 is pushed out of the tube 20, the nucleic acid amplification reaction solution plug 47C flows into the flow path formation portion 35 from the tube. Since the inner diameter of the flow path formation portion 35 is larger than the inner diameter of the capillary 23. Accordingly, the nucleic acid amplification reaction solution plug 47C that has been in the form of a plug (column) inside the tube 20 is formed into a droplet in the flow path formation portion 35. Moreover, since the specific gravity of the nucleic acid amplification reaction solution plug 47C is greater than that of the oil, the nucleic acid amplification reaction solution plug 47C having been formed into a droplet is precipitated.

After the nucleic acid amplification reaction solution plug 47C is pushed out of the tube 20, the oil plug 47B flows into the flow path formation portion 35. After the oil plug 47B is pushed out of the tube 20, the eluate plug 47A flows into the flow path formation portion 35 from the tube 20. Since the inner diameter of the f low path formation portion 35 is larger than the inner diameter of the capillary 23, the eluate plug 47A that has been in the form of a plug (column) inside the tube 20 is formed into a droplet in the flow path formation portion 35. Moreover, the specific gravity of the eluate plug 47A is greater than that of the oil, the eluate plug 47A having been formed into a droplet is precipitated. After being precipitated, the eluate plug 47A having been formed into a droplet mixed with the nucleic acid amplification reaction solution plug 47C, which has already been precipitated to the lower side of the flow path formation portion 35 and is in the form of a droplet, in the oil, whereby the PCR solution 47 in the form of a droplet is formed.

### Description of operation of PCR device 50

The structure of the PCR device 50 of the second embodiment is the same as in the first embodiment. Accordingly, the structure (the rotation mechanism 60, the magnet moving mechanism 70, the pushing mechanism 80, the fluorescence measuring instrument 55, the controller 90, and the like) of the PCR device 50 will not be described again. Herein, the operation of the PCR device 50 on which the cartridge 1 of the second embodiment has been mounted will be described.

### (1) Operation of mounting cartridge 1

The cartridge 1 of the second embodiment is mounted in the same state as described in Figs. 9A to 9D of the first embodiment. In the second embodiment, as shown in Fig. 9C, when the cartridge 1 is fixed to a normal position of the mounting portion 62, the eluate plug 47A of the tube 20 faces the heater for elution 65A (on the contrary, in the first embodiment, the reaction solution plug 47 of the tube 20 faces the heater for elution 65A).

### 2. Nucleic acid elution process

The nucleic acid elution process of the second embodiment is performed in the same state as described in Figs. 11A to 11C of the first embodiment.

In the second embodiment, as shown in Fig. 11B, the magnets 71 move to the position (the section of the eluate plug 47A) facing the eluate plug 47A, and then the controller 90 stops the motor for elevation 73B such that the magnets 71 stop moving in the vertical direction. In this state, the nucleic acid elution process is performed for 30 seconds at 50°C, whereby the nucleic acid having bound to the magnetic beads 7 is liberated into the reaction solution plug 47, and a reverse transcription reaction occurs. By heating of the reaction solution 47, the elution of the nucleic acid from the magnetic beads 7 and reverse transcription are accelerated.

After the nucleic acid is eluted into the eluate plug 47A, the controller 90 drives the motor for elevation 73B in a direction opposite to the direction in which the motor has been driven, such that the carriage 73 slowly moves upward to slowly move the magnets 71 upward. When the magnets 71 move upward in the state described in Fig. 11B, the magnetic beads 7 move to the second oil plug 46 from the eluate plug 47A, whereby the magnetic beads 7 are removed from the eluate plug 47A.

In the second embodiment, the magnetic beads 7 do not come into contact with the nucleic acid amplification reaction solution plug 47C. Accordingly, the enzyme in the nucleic acid amplification reaction solution plug 47C will not be adsorbed onto the magnetic beads 7, and the reaction will not be hindered. Therefore, in the second embodiment, the nucleic acid elution process can be performed under conditions of higher efficiency (higher temperature, higher frequency of oscillation, increase in the amount of magnetic beads, and the like), compared to the first embodiment.

### (3) Solution droplet formation process

The solution droplet formation process of the second embodiment is performed in the same state as described in Figs. 14A to 14C of the first embodiment.

When the plunger 10 is pushed, first, the third oil plug 48 of the tube 20 flows into the flow path formation portion 35, the inflow oil then flows into the oil accommodating space 32A from the flow path formation portion 35, and the oil interface of the oil accommodating space 32A ascends. At this time, due to pressure loss of the lower seal portion 34B, the pressure of the liquid of the flow path formation portion 35 increases.

After the third oil plug 48 is pushed out of the tube 20, the nucleic acid amplification reaction solution plug 47C flows into the flow path formation portion 35 from the tube. Since the inner diameter of the flow path formation portion 35 is larger than the inner diameter of the capillary 23, the nucleic acid amplification reaction solution plug 47C which has been in the form of a plug (column) inside the tube 20 is formed into a droplet in the flow path formation portion 35. Moreover, since the specific gravity of the nucleic acid amplification reaction solution plug 47C is greater than that of the oil, the nucleic acid amplification reaction solution plug 47C having been formed into a droplet is precipitated.

After the nucleic acid amplification reaction solution plug 47C is pushed out of the tube 20, the oil plug 47B flows into the flow path formation portion 35. After the oil plug 47B is pushed out of the tube 20, the eluate plug 47A flows into the flow path formation portion 35 from the tube 20. Since the inner diameter of the flow path formation portion 35 is larger than the inner diameter of the capillary 23, the eluate plug 47A that has been in the form of a plug (column) inside the tube 20 is formed into a droplet in the flow path formation portion 35. Moreover, the specific gravity of the eluate plug 47A is greater than the oil, the eluate plug 47A having been formed into a droplet is precipitated. After being precipitated, the eluate plug 47A having been formed into a droplet is mixed with the nucleic acid amplification reaction solution plug 47C, which has already been precipitated to the lower side of the flow path formation portion 35 and is in the form of a droplet, in the oil, whereby the PCR solution 47 in the form of a droplet is formed.

Droplets of both the eluate plug 47A and the nucleic acid amplification reaction solution plug 47C are small. Accordingly, sometimes the two droplets remain separated from each other without being mixed with each other in the lower side of the flow path formation portion 35. Therefore, after the eluate plug 47A in the form of a droplet is precipitated to the lower side of the flow path formation portion 35, the controller 90 drives the rotation mechanism 60 little by little to cause the rotary body 61 to oscillate. As a result, even if the two droplets are in a state of being separated from each other in the lower side of the flow path formation portion 35, by the oscillation of the cartridge 1, the two droplets are mixed with each other, whereby the PCR solution 47 in the form of a droplet is formed. Moreover, the droplets may be heated by the high temperature-side heater 65B or the low temperature-side heater 65C so as to decrease viscosity of the droplets and to make it easy for the two droplets to be mixed with each other.

After the solution droplet formation process, the thermal cycling process or fluorescence measurement is performed. Since the thermal cycling process and fluorescence measurement of the second embodiment are performed in the same manner as in the first embodiment, they will not be described herein.

In the second embodiment, the PCR device 50 also has the rotary body 61 that includes a mounting portion (consisting of the fixing portion 63 and the insertion hole 64A) on which the cartridge 1 is mounted, and heaters for PCR (the high temperature-side heater 65B and the low temperature-side heater 65C) that forms a temperature gradient in the inside of the PCR container 30 as a nucleic acid amplification reaction container. Moreover, as the posture of the cartridge 1 is changed due to the rotation of the rotary body 61, the reaction solution 47 in the form of a droplet which has been introduced from the tube 20 moves inside the PCR container 30. As a result, the posture of the tube 20, in which the nucleic acid elution process is performed, and the posture of the PCR container 30 can be changed at the same time to perform the polymerase reaction. Consequently, the process time can be shortened.

### Other aspects

The above embodiments are for making the invention easily understandable, and the invention is not limited to those embodiments. Needless to say, the invention can be modified or improved within a range that does not depart from the gist of the invention, and the invention also includes those obtained by the modification and improvement.

### Regarding PCR device

The PCR device 50 changes the posture of the cartridge 1 by predetermined cycle numbers to perform thermal cycling process and makes the PCR container 30 become upside down. However, the posture of the cartridge 1 does not have to be changed plural times and may be changed once.

In the aforementioned PCR device, the rotary shaft of the rotary body is positioned closer to the tube than to the PCR container. However, as long as the solution droplet can move inside the PCR container when the posture of the cartridge is changed due to the rotation of the rotary body, the position of the rotary shaft of the rotary body is not limited to the above position.

In the aforementioned PCR device, the fixing portion 63, in which a notch has been formed, and the insertion hole 64A constitutes the mounting portion of the cartridge. However, the mounting portion is not limited to this constitution, as long as the cartridge can be mounted on the rotary body. The mounting portion may have a structure in which the cartridge is fixed to the rotary body by only fixing the tube side, or may have a structure in which the cartridge is fixed to the rotary body by only fixing the PCR container side. However, the mounting portion needs to have a structure in which the cartridge is stably fixed to the rotary body even if the posture of the cartridge is changed due to the rotation of the rotary body.

The aforementioned PCR device 50 has the high temperature-side heater 65B and the low temperature-side heater 65C as heaters for PCR. However, the device is not limited to this constitution as long as a temperature gradient can be formed inside the PCR container as a nucleic acid amplification reaction container. For example, the heater may be disposed only at a high-temperature side. Alternatively, a heater may be disposed at a high-temperature side, and a cooler may be disposed at a low-temperature side.

In the aforementioned PCR device 50, heaters for PCR (the high temperature-side heater 65B and the low temperature-side heater 65C) are disposed in the rotary body. However, the heaters for PCR may be disposed in the outside of the rotary body, as long as a temperature gradient can be formed inside the PCR container as a nucleic acid amplification reaction container. For example, a first heater for PCR, which faces the PCR container when the rotary body 61 is in the standard position as shown in FIG. 15A, and a second heater for PCR, which faces the PCR container when the rotary body rotates by 180° as shown in Fig. 15B, may be disposed in the outside of the rotary body of the PCR device. Even in this structure, a temperature gradient can be formed inside the PCR container as a nucleic acid amplification reaction container. However, it is desirable for the heaters for PCR to be disposed in the rotary body, since the positional relationship between the PCR container of the cartridge and the heaters can be maintained regardless of the rotation position of the rotary body.

The PCR device 50 may only have the heaters for PCR (for example, the high temperature-side heater 65B and the low temperature-side heater 65C) without the heater for elution 65A. However, it is desirable for the PCR device 50 to have the heater for elution 65A since the liberation of nucleic acid from magnetic beads is accelerated.

The PCR device 50 may not have the magnet moving mechanism that moves magnets along the tube. In this case, for example, the operator may grab and move the magnets along the tube. However, it is desirable for the PCR device 50 to have the magnet moving mechanism, since the movement speed of the magnetic beads as nucleic acid-binding solid-phase carriers may be changed because of the operator.

The magnet moving mechanism 70 of the PCR device 50 may not have the oscillation mechanism 75. In this case, though the magnets cannot be caused to oscillate, they can be moved along the tube. Accordingly, the magnetic beads having bound to nucleic acid can be moved to the plug containing the eluate.

The PCR device 50 may not have the pushing mechanism 80. In this case, for example, the operator may manually push the plunger of the cartridge. When the plunger is not disposed in the cartridge, the operator may deform the tank to increase the internal pressure of the tank such that the liquid is pushed out to the PCR container from the tube.

The PCR device 50 may not have the fluorescence measuring instrument. In this case, the device cannot perform real time PCR, but it can perform a polymerase reaction for amplifying nucleic acid.

### Regarding eluate plug 47A

The eluate plug 47A may be divided into a reverse transcription reaction solution plug and an eluate plug. In this case, the nucleic acid-binding solid-phase carriers having been washed with the washing solution is moved to the reverse transcription reaction solution plug to cause a reverse transcription reaction in the reverse transcription reaction plug. This reaction can be conducted under conditions suitable for the used reverse transcriptase. For example, the reverse transcription reaction solution is heated to 30°C to 50°C, preferably 42°C to 45°C, and the nucleic acid-binding solid-phase carriers are held in the solution for a predetermined time, whereby the reverse transcription reaction can be caused in a state where RNA is being bonded to the carriers. The heating method is not particularly limited. For example, a method of bringing a heat medium such as a heat block into contact with a position facing the reverse transcription reaction solution plug of the tube, a method of using heat source such as a heater, a method of performing electromagnetic heating, and the like can be used. The holding time can also be appropriately selected by the operator. However, the carriers may be held for 10 seconds to 5 minutes, and preferably for 30 seconds to 1 minute. cDNA synthesized at this stage is bonded to the solid-phase carriers in a state of being bonded to RNA.

Thereafter, the nucleic acid-binding solid-phase carriers are moved to the eluate plug. At this time, in order to cause the nucleic acid, particularly cDNA to be efficiently liberated from the nucleic acid-binding solid-phase carriers, it is preferable to heat the eluate plug. The heating method is not particularly limited, and the same methods as being used for heating the reverse transcription reaction solution plug can be used. The heating temperature may be higher than 40°C, and is preferably 50°C or higher and more preferably 60°C or higher. The upper limit of the heating temperature is not particularly limited. However, the upper limit is preferably 70°C or lower, more preferably 65°C or lower, even more preferably 60°C or lower, and most preferably 60°C.

After the nucleic acid is liberated from the nucleic acid-binding solid-phase carriers, the nucleic acid-binding solid-phase carriers are moved upward from the eluate plug by using magnets. The nucleic acid-binding solid-phase carriers may be moved to any position as long as the nucleic acid-binding solid-phase carriers are not mixed into the eluate plug.

If the eluate plug is divided into a reverse transcription reaction solution plug and an eluate plug as described above, each of reverse transcription reaction and nucleic acid elution can be performed under conditions brining high efficiency.

### Examples

### Experiment example

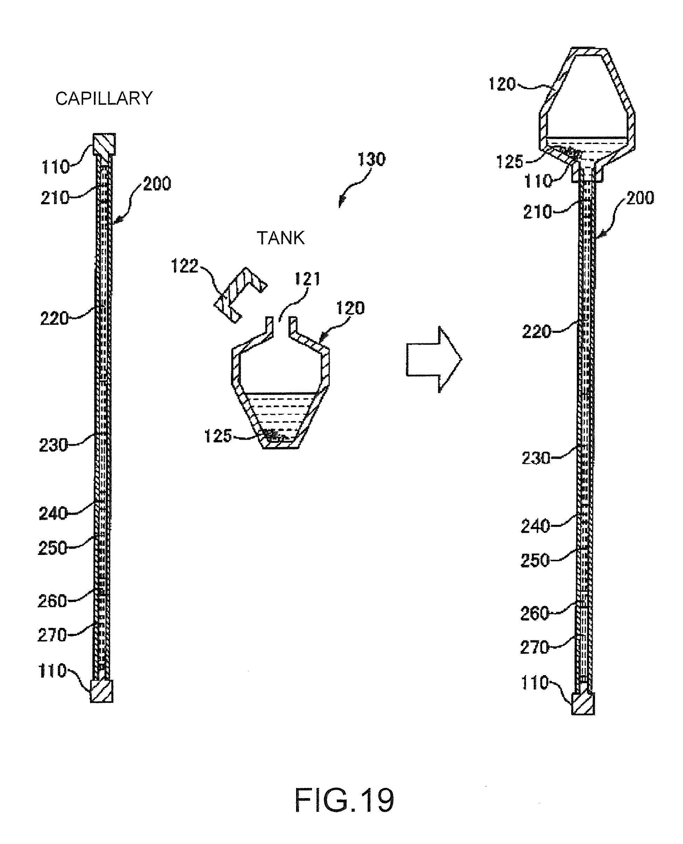

In the present experiment example, among the nucleic acid extraction kits described above, constitution in which a first plug 210 to a seventh plug 270 are contained in a tube 200 as shown in Fig. 19 was used.

First, 375 µL of an adsorbent solution and 1 µL of magnetic beads dispersion were put in a polyethylene container 130 with a capacity of 3 mL. As the adsorbent solution, an aqueous solution containing 76% by mass of guanidine hydrochloride, 1.7% by mass of ethylenediaminetetraacetic acid disodium salt dehydrate, and 10% by mass of polyoxyethylene sorbitan monolaurate (manufactured by TOYOBO CO., LTD., MagExtractor-Genome, NPK-1) was used. Moreover, as a magnetic beads solution, a solution containing 50% by volume of magnetic silica particles and 20% by mass of lithium chloride was used.

50 µL of the blood collected from a human being was put in a container 130 from an opening 121 by using a pipette, the container 130 was covered with a lid 122, and the container was manually shaken for 30 seconds to stir the content in the container. Thereafter, the lid 122 of the container 130 was removed, and the container was connected to the tube 200. Note that both ends of the tube 200 had been sealed with a stopper 110. The stopper 110 at the side of the first plug 210 was removed, and the container 130 was connected to the tube 200.

Silicon oil was used as the first plug 210, the third plug 230, the seventh plug 270, and the fifth plug 250. For a first washing solution as the second plug 220, an aqueous solution of 76% by mass of guanidine hydrochloride was used. Moreover, for a second washing solution as the fourth plug 240, Tris-HCl buffer (solute concentration of 5 mM) with pH of 8.0 was used. For the eluate as the sixth plug 260, sterile water was used.

Subsequently, a permanent magnet 410 was moved to introduce magnetic beads 125 in the container 130 into the tube 200. Then the magnetic beads 125 were moved to the sixth plug 260. The time during which the magnetic beads 125 stayed in each plug in the tube 200 is as follows; the first, third, and seventh plugs: 3 seconds, the second plug: 20 seconds, the fourth plug: 20 seconds, the sixth plug: 30 seconds. In the second plug 220 and the fourth plug 240, operation for causing the magnetic beads to oscillate was not performed. Moreover, the volume of the second plug 220, the fourth plug 240, and the sixth plug 260 were 25 µL, 25 µL, and 1 µL respectively.

Thereafter, the stopper 110 at the side of the seventh plug of the tube was removed, and the container 120 was manually deformed such that the seventh plug 270 and the sixth plug 260 were ejected into the PCR reaction container. This operation was performed after the magnetic beads were moved by using the permanent magnet to cause the magnetic beads retract to the second plug 220.

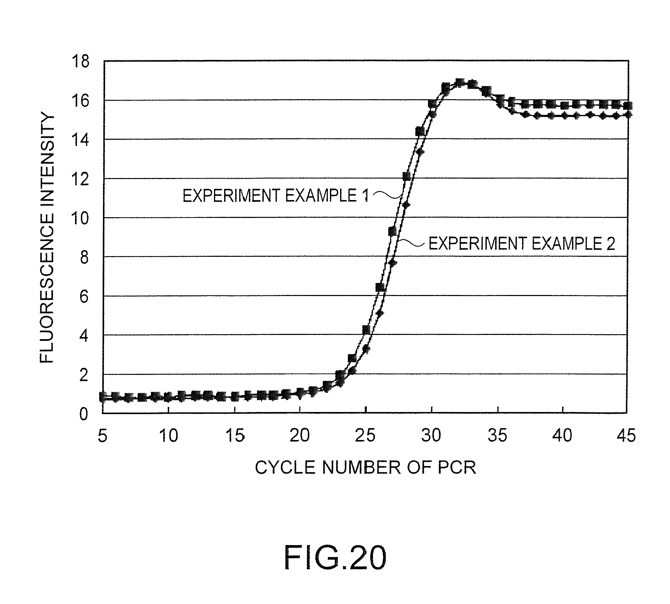

Then 19 µL of a PCR reaction reagent was added to the extract obtained as above to perform real time PCR according to the usual method. The PCR reaction reagents were composed of 4 µL of LightCycler 480 Genotyping Master (manufactured by Roche Diagnostics, 4 707 524), 0.4 µL of SYBR Green I (manufactured by Life Technologies Corporation., S7563) diluted with sterile water by 1,000-fold, 0.06 µL of 100 µM primers (F/R) for detecting β actin, and 14.48 µL of sterile water. Fig. 20 shows a PCR amplification curve of Experiment example 1. In Fig. 20, the ordinate indicates fluorescence intensity, and the abscissa indicates the cycle number of PCR.

### Experiment example 2

In Experiment example 2, nucleic acid was extracted by a general nucleic acid extraction method.

First, 375 µL of an adsorbent solution and 20 µL of magnetic beads dispersion were put in a polyethylene container (Eppendorf tube) with a capacity of 1.5 µL. The composition of the adsorbent solution and magnetic beads dispersion was the same as in the above experiment example.

50 µL of the blood collected from a human being was put in the container from the opening thereof by using a pipette, and the container was covered with a lid. Then the content of the container was stirred for 10 minutes by using a vortex mixer, and B/F separation operation was performed by using a magnetic stand and a pipette. In this state, the magnetic beads and a small amount of the adsorbent solution remained in the container.

Subsequently, 450 µL of the first washing solution having the same composition as in Experiment example 1 was put into the container, the container was covered with a lid, the content in the container was stirred for 5 seconds with a vortex mixer, and then the first washing solution was removed by using a magnetic stand and a pipette. This operation was repeated twice. In this state, the magnetic beads and a small amount of the first washing solution remained in the container.

Then 450 µL of the second washing solution having the same composition as in Experiment example 1 was put into the container, the container was covered with a lid, the content in the container was stirred for 5 seconds with a vortex mixer, and the second washing solution was removed by using a magnetic stand and a pipette. This operation was repeated twice. In this state, the magnetic beads and a small amount of the second washing solution remained in the container.

Subsequently, 50 µL of sterile water (eluate) was added to the container, the container was covered with a lid, and the content in the container was stirred with a vortex mixer for 10 minutes, and the supernatant liquid was collected by using a magnetic stand and a pipette. The supernatant liquid contained the target nucleic acid.

Thereafter, 1 µL of the extract obtained as above was dispensed, and 19 µL of PCR reaction reagent was added thereto to perform real time PCR according to the usual method. The PCR reaction reagent was composed of 4 µL of LightCycler 480 Genotyping Master (manufactured by Roche Diagnostics, 4 707 524), 0.4 µL of SYBR Green I (manufactured by Life Technologies Corporation., S7563) diluted with sterile water by 1,000-fold, 0.06 µL of 100 µM primers (F/R) for detecting P actin, and 14.48 µL of sterile water. Fig. 20 shows a PCR amplification curve obtained at this time.

### Experiment results

From the above Experiment examples, the following can be understood.

(1) The Experiment examples were compared to each other in terms of the time taken for the nucleic acid extraction process which is pre-process of PCR. As a result, it was found that the time from when the sample is put into the container to when the target nucleic acid is put into the PCR reaction container is about 2 minutes in Experiment example 1 and about 30 minutes in Experiment example 2. This shows that the time taken for extracting the nucleic acid is significantly shortened in the nucleic acid extraction method of Experiment example 1, compared to the nucleic acid extraction method of Experiment example 2.

(2) The amount of the respective washing solutions of Experiment example 1 was about one eighteenth of that of Experiment example 2. Moreover, the amount of the eluate of Experiment example 1 was about one fiftieth of that of Experiment example 2. Accordingly, it is understood that Experiment example 1 uses an extremely small amount of the washing solutions and eluate compared to Experiment example 2.

(3) If the experiment examples are compared to each other in terms of the concentration of the target nucleic acid in the eluate based on the amount of the adsorbent solution and the eluate, ideally, the concentration of Experiment example 1 will be 50 times higher than that of Experiment example 2. However, in the present experiment examples, the amount of nucleic acid contained in the blood sample was as large as exceeding the amount of nucleic acid that 1 µL of magnetic beads can adsorb, and the entire nucleic acid contained in the blood sample could not be collected. Accordingly, the concentration of nucleic acid of Experiment example 1 was not 50 times higher than that of Experiment example 2. When a sample containing nucleic acid in such an amount that does not exceed the amount of nucleic acid which can be adsorbed onto at least 1 µL of magnetic beads is used, the concentration of nucleic acid of Experiment example 1 can be 50 times higher than that of Experiment example 2.

(4) As shown in the graph of Fig. 20, even in the whole blood sample containing a large amount of nucleic acid, the nucleic acid amplification rate is higher in Experiment example 1 than in Experiment example 2 by about 0.6 cycles. That is, the concentration of the target nucleic acid is higher in the PCR reaction solution used in Experiment example 1 than in the PCR reaction solution used in Experiment example 2. In other words, the concentration of the target nucleic acid in the eluate is higher in Experiment example 1 than in Experiment example 2.

## Claims

1. A nucleic acid amplification reaction device comprising:
a rotary body having a mounting portion on which a cartridge, which includes a tube that has a plug containing an eluate in which a nucleic acid having bound to nucleic acid-binding solid-phase carriers is eluted from the carriers and a nucleic acid amplification reaction container that is in communication with the tube and contains oil undergoing phase separation from the eluate, is configured to be mounted; and
a heater forming a temperature gradient inside the nucleic acid amplification reaction container,
wherein when the posture of the cartridge is changed due to the rotation of the rotary body, a droplet of the eluate introduced from the tube moves inside the nucleic acid amplification reaction container.

2. The nucleic acid amplification reaction device according to claim 1,
wherein a rotary shaft of the rotary body is positioned not in the nucleic acid amplification reaction container but at the side of the tube.

3. The nucleic acid amplification reaction device according to claim 1 or 2,
wherein the mounting portion includes a tube fixing portion to which the tube is fixed and a container fixing portion to which the nucleic acid amplification reaction container is fixed.

4. The nucleic acid amplification reaction device according to any one of claims 1 to 3,
wherein the heater is disposed in the rotary body.

5. The nucleic acid amplification reaction device according to any one of claims 1 to 4, further comprising a heater for elution that is configured to heat the plug of the cartridge mounted on the mounting portion.

6. The nucleic acid amplification reaction device according to any one of claims 1 to 5, further comprising a magnet moving mechanism that is configured to move magnets along the tube.

7. The nucleic acid amplification reaction device according to claim 6,
wherein the magnet moving mechanism is configured to cause the magnets to oscillate such that a distance between the magnets and the tube is changed.

8. The nucleic acid amplification reaction device according to any one of claims 1 to 7, further comprising a pushing mechanism that is configured to push a plunger disposed in the cartridge such that liquid is pushed to the nucleic acid amplification reaction container out of the tube.

9. A nucleic acid amplification method comprising:
mounting a cartridge, which includes a tube that has a plug containing an eluate in which a nucleic acid having bound to nucleic acid-binding solid-phase carriers is eluted from the carriers and a nucleic acid amplification reaction container that is in communication with the tube and contains oil undergoing phase separation from the eluate, on a mounting portion; and
forming a temperature gradient inside the nucleic acid amplification reaction container and changing the posture of the cartridge by causing a rotary body having the mounting portion to rotate such that a droplet of the eluate introduced from the tube moves inside the nucleic acid amplification reaction container.
